# EUROPEAN PATENT APPLICATION

(11) **EP 4 635 400 A1**
(43) Date of publication of application: **22.10.2025**
(21) Application number: 25169734.8
(22) Date of filing: 10.04.2025
(51) Int. Cl.: A61B 3/10, A61B 3/12, A61B 3/15

(54) **OPHTHALMIC APPARATUS**

(30) Priority: 16.04.2024 JP 2024066363
(71) Applicant: TOPCON CORPORATION, Tokyo 174-8580 (JP)
(72) Inventor: NAKAJIMA, Masashi, Tokyo, 174-8580 (JP); MORISHIMA, Syunichi, Tokyo, 174-8580 (JP); KAWARAI, Makoto, Tokyo, 174-8580 (JP)
(74) Representative: Gagel, Roland

(57) **Abstract**

An ophthalmic apparatus includes an objective optical system, an illumination optical system, a photographic optical system, a photographing unit, a movement mechanism, and a three-dimensional position identifying unit. The objective optical system is configured to optically relay a measurement position where a pupil of an eye of an examinee can be disposed. The illumination optical system is configured to irradiate illumination light onto the eye through the objective optical system. The photographic optical system is configured to receive returning light of the illumination light from the eye through the objective optical system. The photographing unit includes two or more cameras disposed so as to include a position, that is substantially conjugate optically to the measurement position relayed by the objective optical system, within a field of vision. The movement mechanism is configured to relatively move the objective optical system, the illumination optical system, the photographic optical system, and the photographing unit relative to the eye. The three-dimensional position identifying unit is configured to identify a three-dimensional position of the eye based on two or more photographic images of the eye acquired by the photographing unit.

## Description

### FIELD

The disclosure relates to an ophthalmic apparatus.

### BACKGROUND

Ophthalmic apparatuses used for screening or treatment of eye diseases are expected to be capable of easily photographing (observing) a fundus of an eye to be examined with a wide field of view. Specifically, the ophthalmic apparatuses capable of photographing the fundus of the eye to be examined at a wide photographing (shooting) angle of view of more than 80 degrees in a single photographing (shot) are expected. As such ophthalmic apparatuses, scanning laser ophthalmoscopes (SLOs) are known. SLO is an apparatus configured to form an image of the fundus by scanning the fundus with light to detect returning light of the light with a light receiving device.

Japanese Unexamined Patent Application Publication (Translation of PCT Application) No. 2009-543585 discloses a scanning ophthalmoscope that can scan a retina at a wide angle by moving a two-dimensional collimated optical scan using a polygon mirror and a plane mirror onto the eye to be examined using a scan moving means.

U.S. Patent No. 7,831,106 and WO 2022/124170 disclose a fundus photographing apparatus that can acquire high-contrast images with a simple configuration, by combining a fundus scanning using slit-shaped illumination light and a rolling shutter system. In particular, WO 2022/124170 discloses a method of acquiring wide-angle fundus images of the eye to be examined by scanning the fundus of the eye to be examined with the slit-shaped illumination light through two ellipsoidal concave mirrors.

In such ophthalmic apparatuses, the use of a curved mirror such as an ellipsoidal concave mirror leads to enlargement of the apparatus. Thus, reducing the curvature of the curved mirror can be considered as a way to downsize the apparatus. However, in addition to the need to shorten the distance between the curved mirror and the eye to be examined, the eye to be examined must be precisely aligned with an optical system of the apparatus.

Japanese Unexamined Patent Application Publication No. 2019-062981 discloses a method for providing two photographing units in a direction deflected by a deflecting member disposed facing the eye to be examined, and for aligning the optical system of apparatus with the eye to be examined by using two photographic images acquired using the two photographing units.

### SUMMARY

One aspect of some embodiments is an ophthalmic apparatus including: an objective optical system configured to optically relay a measurement position where a pupil of an eye of an examinee can be disposed; an illumination optical system configured to irradiate illumination light onto the eye through the objective optical system; a photographic optical system configured to receive returning light of the illumination light from the eye through the objective optical system; a photographing unit including two or more cameras disposed so as to include a position, that is substantially conjugate optically to the measurement position relayed by the objective optical system, within a field of vision; a movement mechanism configured to relatively move the objective optical system, the illumination optical system, the photographic optical system, and the photographing unit relative to the eye; and a three-dimensional position identifying unit configured to identify a three-dimensional position of the eye based on two or more photographic images of the eye acquired by the photographing unit.

### BRIEF DESCRIPTION OF THE DRAWINGS

FIG. 1 is a schematic diagram illustrating an example of a configuration of an optical system of an ophthalmic apparatus according to embodiments.
FIG. 2 is a schematic diagram for explaining the configuration of the optical system of the ophthalmic apparatus according to the embodiments.
FIG. 3A is a schematic diagram for explaining the configuration of the optical system of the ophthalmic apparatus according to the embodiments.
FIG. 3B is a schematic diagram for explaining the configuration of the optical system of the ophthalmic apparatus according to the embodiments.
FIG. 4A is a schematic diagram for explaining the configuration of the optical system of the ophthalmic apparatus according to the embodiments.
FIG. 4B is a schematic diagram for explaining the configuration of the optical system of the ophthalmic apparatus according to the embodiments.
FIG. 5 is a schematic diagram for explaining the configuration of the optical system of the ophthalmic apparatus according to the embodiments.
FIG. 6A is a schematic diagram for explaining the configuration of the optical system of the ophthalmic apparatus according to the embodiments.
FIG. 6B is a schematic diagram for explaining the configuration of the optical system of the ophthalmic apparatus according to the embodiments.
FIG. 7 is a schematic diagram illustrating an example of a configuration of the optical system of the ophthalmic apparatus according to the embodiments.
FIG. 8 is a schematic diagram illustrating an example of a configuration of the optical system of the ophthalmic apparatus according to the embodiments.
FIG. 9 is a schematic diagram illustrating an example of a configuration of the optical system of the ophthalmic apparatus according to the embodiments.
FIG. 10 is a schematic diagram for explaining the configuration of the optical system of the ophthalmic apparatus according to the embodiments.
FIG. 11 is a schematic diagram for explaining the configuration of the optical system of the ophthalmic apparatus according to the embodiments.
FIG. 12 is a schematic diagram illustrating an example of a configuration of the optical system of the ophthalmic apparatus according to the embodiments.
FIG. 13 is a schematic diagram for explaining the configuration of the optical system of the ophthalmic apparatus according to the embodiments.
FIG. 14 is a schematic diagram illustrating an example of a configuration of a control system of the ophthalmic apparatus according to the embodiments.
FIG. 15 is a schematic diagram illustrating an example of a configuration of the control system of the ophthalmic apparatus according to the embodiments.
FIG. 16 is a flowchart illustrating an example of an operation of the ophthalmic apparatus according to the embodiments.
FIG. 17 is a flowchart illustrating an example of an operation of the ophthalmic apparatus according to the embodiments.
FIG. 18 is a flowchart illustrating an example of an operation of the ophthalmic apparatus according to the embodiments.
FIG. 19 is a schematic diagram for explaining an operation of the ophthalmic apparatus according to the embodiments.

### DETAILED DESCRIPTION

When the curvature of the curved mirror such as an ellipsoidal concave mirror is reduced to shorten the distance between the curved mirror and the eye to be examined, it becomes difficult to dispose an alignment optical system that enables high-precision positioning matching.

The situation described above is the same not only when the objective optical system facing the eye to be examined is a reflective optical system including a curved mirror, etc., but also when the objective optical system is a refractive optical system including an objective lens, etc.

According to some embodiments of the present invention, a new technique for photographing or measuring an eye to be examined at a wide angle while preferably performing position matching between an optical system of apparatus and the eye to be examined can be provided.

Referring now to the drawings, exemplary embodiments of an ophthalmic apparatus according to the present invention are described below. Any of the contents of the documents cited in the present specification and arbitrary known techniques may be applied to the embodiments below.

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

An ophthalmic apparatus according to embodiments is configured to irradiate light onto an eye to be examined at a wide angle using an irradiation optical system through an objective optical system provided so as to face the eye to be examined (eye of an examinee). Furthermore, the ophthalmic apparatus is configured to photograph or measure the eye to be examined by receiving returning light from the eye to be examined using a light receiving optical system. In case of photographing the eye to be examined, the irradiation optical system is an illumination optical system configured to irradiate illumination light onto the eye to be examined, and the light receiving optical system is a photographic optical system configured to receive the returning light of the illumination light from the eye to be examined. By photographing the eye to be examined at a wide angle, wide-angle images of the eye to be examined can be acquired. By measuring the eye to be examined at a wide angle, measured values of optical characteristics of a wide range of the eye to be examined can be acquired.

In addition, the ophthalmic apparatus according to the embodiments includes a movement mechanism configured to relatively move an optical system of apparatus relative to the eye to be examined, and is configured to be capable of performing position matching between the eye to be examined and the optical system of apparatus.

Specifically, the objective optical system is configured to optically relay a measurement position where the eye to be examined can be disposed. In some embodiments, the objective optical system includes a reflective optical system including a reflective member (for example, a curved mirror) disposed facing the eye to be examined. In some embodiments, the objective optical system includes a refractive optical system including a refractive member (for example, an objective lens) disposed facing the eye to be examined. The ophthalmic apparatus includes a photographing unit (shooting unit, imaging unit) having two or more cameras disposed so as to include a measurement conjugate position, that is substantially conjugate optically to the measurement position, that is described above, relayed by the objective optical system, within a field of vision. In other words, each of the two or more cameras is disposed to optically view (see) the measurement conjugate position. In this case, the ophthalmic apparatus can include a first optical path separating member (separator) and a second optical path separating member (separator) . The first optical path separating member is disposed at the measurement conjugate position, and is configured to separate an optical path of light from the irradiation optical system (illumination optical system) and an optical path of returning light from the eye to be examined. The second optical path separating member is disposed between the objective optical system and the first optical path separating member, and is configured to guide at least part of light from the eye to the photographing unit.

Furthermore, the ophthalmic apparatus is configured to identify a three-dimensional position of the eye to be examined based on two or more photographic images of the eye to be examined acquired using the two or more cameras. For example, the two or more cameras are disposed so that angles formed by a measurement conjugate plane (plane perpendicular to an optical axis of an optical system of the photographing unit) at a position substantially conjugate optically to the measurement position described above and photographic optical axes of the cameras are the same, and that the angles are symmetrical with reference to a normal direction of the measurement conjugate plane. In some embodiments, each of the two or more cameras is disposed on each of two or more photographic optical axes, each of the photographic optical axes being intersecting a photographic reference optical axis passing through the measurement conjugate position relayed by the optical system. Here, a pupil (pupil region) of the eye to be examined can be disposed at the measurement position. In this case, the ophthalmic apparatus can identify a three-dimensional position of the pupil of the eye to be examined, as the three-dimensional position of the eye to be examined, based on two or more anterior segment images of the eye to be examined acquired using the two or more cameras.

In some embodiments, the ophthalmic apparatus is configured to be capable of performing position matching of the optical system of apparatus with respect to the eye to be examined, by manually moving the optical system of apparatus relative to the eye to be examined by an examiner or the examinee using a movement mechanism, based on the identified three-dimensional position of the eye to be examined (pupil). The movement mechanism is configured to relatively move the optical system of apparatus relative to the eye to be examined, by three-dimensionally moving the optical system (objective optical system, illumination optical system, photographic optical system, and photographing unit) of the apparatus.

In some embodiments, the ophthalmic apparatus is configured to be capable of performing position matching of the optical system of apparatus with respect to the eye to be examined, by moving the optical system of apparatus relative to the eye to be examined by a controller controlling a movement mechanism based on the three-dimensional position of the eye to be examined (pupil).

Thereby, even when a distance between the eye to be examined and the objective optical system is short, the two or more photographic images of the eye to be examined can be acquired, and the position matching between the optical system of the apparatus and the eye to be examined can be preferably performed based on the acquired two or more photographic images. As a result, high-definition photographing (shooting, imaging) or high-precision measurement of the eye to be examined at a wide angle can be performed.

Hereinafter, it is assumed that the ophthalmic apparatus according to the embodiments is a fundus photographing apparatus. In this case, the fundus photographing apparatus includes an objective optical system with two or more curved mirrors, illuminates a fundus of the eye to be examined using slit scanning system, and photographs the fundus at a wide angle by receiving returning light of the illumination light from the fundus. In other words, the ophthalmic apparatus is configured to scan the fundus of the eye to be examined with slit-shaped illumination light through the two or more curved mirrors, and to receive the returning light from the fundus through the two or more curved mirrors using an image sensor. In this case, the ophthalmic apparatus includes a slit, in which an aperture (opening) is formed, the aperture being configured to be disposed at a fundus conjugate position substantially conjugate optically to the fundus, and generates the slit-shaped illumination light by irradiating light from a light source onto the slit. Further, the image sensor is configured to be disposed at the fundus conjugate position.

It should be noted that the embodiments are not limited to ophthalmic apparatus (fundus photographing apparatus) for photographing the fundus, but can be applied to ophthalmic apparatus (fundus observation apparatus) for observing the fundus. Further, the following embodiments can also be applied to ophthalmic apparatus for photographing or observing a site other than the fundus of the eye to be examined. Furthermore, the following embodiments can be applied to ophthalmic apparatus for measuring the fundus or a site other than the fundus of the eye to be examined.

In some embodiments, each of the two or more curved mirrors has one or more focal points, and the two or more curved mirrors are arranged so as to share at least one of the focal points. For example, the two or more curved mirrors are arranged so that two or more focal points of the two or more curved mirrors are disposed on an approximately same plane (common plane of focal points).

For example, the ophthalmic apparatus is configured to illuminate the fundus with the illumination light so that a longitudinal direction of a slit image formed by the illumination light projected onto the fundus is approximately parallel to a plane including the two or more focal points, and to scan the fundus with the illumination light in a direction intersecting the longitudinal direction. Specifically, the ophthalmic apparatus is configured to scan the fundus with illumination light in a direction perpendicular to the longitudinal direction of the slit image. Here, the slit image is an image of the aperture formed in the slit.

Examples of the curved mirror include an ellipsoidal mirror, a paraboloidal mirror, a hyperboloidal mirror, a free-form surface mirror, and a mirror whose reflective surface is represented by higher-order polynomials. The reflective surface of the curved mirror may be a concave-shaped reflective surface or a convex-shaped reflective surface. In this case, examples of the curved mirror include an ellipsoidal concave mirror, an ellipsoidal convex mirror, a paraboloidal concave mirror, a paraboloidal convex mirror, a hyperboloidal concave mirror, a hyperboloidal convex mirror, a free-form surface mirror having a concave-shaped reflective surface, a free-form surface mirror having a convex-shaped reflective surface, a concave mirror whose reflective surface is represented by higher-order polynomials. and a convex mirror whose reflective surface is represented by higher-order polynomials.

In this specification, the focal point may include not only a fixed point uniquely determined by the shape of the curved surface, but also a position where the light beams (light fluxes) reflected by the reflective surface are more focused than at other positions. Furthermore, the position of the pupil and the position of the iris of the eye to be examined are sometimes described as being in the same position.

The common plane of focal points is preferably a plane on which all focal points of the two or more curved mirrors are disposed. However, the common plane of focal points may be a plane on which two or more focal points, which are obtained by excluding at least one of all the focal points that the two or more curved mirrors have, are disposed.

A method of controlling the ophthalmic apparatus according to the embodiments includes one or more steps for realizing the processing executed by a processor (computer) in the ophthalmic apparatus according to the embodiments. A program according to the embodiments causes the processor to execute each step of the method of controlling the ophthalmic apparatus according to the embodiments. In other words, the program according to the embodiments is a computer program comprising instructions which, when the program is executed by a computer, cause the computer to carry out the method of controlling the ophthalmic apparatus according to the embodiments. A recording medium (storage medium) according to the embodiments is any computer readable non-transitory recording medium (storage medium) on which the program according to the embodiments is recorded. The recording medium may be an electronic medium using magnetism, light, magneto-optical, semiconductor, or the like. Typically, the recording medium is a magnetic tape, a magnetic disk, an optical disk, a magneto-optical disk, a flash memory, a solid state drive, or the like. Examples of magnetic disk include a magnetic storage media such as a hard disk, a floppy (registered trademark) disks, and a ZIP. Examples of the magneto-optical disk include CD-ROM, DVD-RAM, DVD-ROM, and MO. The program may be transmitted and received through a network such as the Internet, LAN, etc.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, a case where the ophthalmic apparatus according to the embodiments includes two or more ellipsoidal concave mirrors as the two or more curved mirrors will be mainly described. However, the following embodiments can be applied to the ophthalmic apparatus including three or more curved mirrors.

Hereinafter, for convenience of explanation, a depth direction (front-back direction) of the apparatus is assumed to be a Z direction, a horizontal direction (left-right direction) perpendicular to the Z direction is assumed to be an X direction, and a vertical direction (up-down direction) perpendicular to the Z direction is assumed to be a Y direction. In some embodiments, the Z direction is an optical axis direction of the illumination light entering the eye to be examined. Here, among the Z directions, a direction that approaches the eye to be examined eye may be referred to as "+Z direction", and a direction that moves away from the eye to be examined may be referred to as "-Z direction". Further, among the X directions, a direction from the left eye to the right eye of the examinee may be referred to as "+X direction", and a direction from the right eye to the left eye of the examinee may be referred to as "-X direction". Furthermore, among the Y directions, a direction from the eye to the forehead (upward direction) may be referred to as "+ Y direction", and a direction from the forehead to the eye (downward direction) may be referred to as "-Y direction".

Furthermore, hereinafter, "longitudinal direction" of the slit image is assumed to be meant to be a direction in which the long side of the rectangle that circumscribes the slit image extends (longer direction), and "shorter direction" of the slit image is assumed to be meant to be a direction in which the short edge of the rectangle circumscribed by the slit image extends (widthwise direction, shortitudinal direction).

### <Optical system>

In the ophthalmic apparatus according to the embodiments, two ellipsoidal concave mirrors are arranged so that the major axis directions of both of the two ellipsoidal concave mirrors are approximately parallel to an arrangement direction of the left eye (left eye to be examined) and the right eye (right eye to be examined) of the examinee to be photographed at the time of photography. Thereby, the eye to be examined can be photographed at a wide angle in a state where a distance between the eye to be examined and the ellipsoidal concave mirror is brought closer without interfering with the face of the examinee. Such an ophthalmic apparatus is configured to photograph the fundus sequentially for the left and right eyes of the examinee.

In the present embodiment, in case that the illumination light, which is deflected over a wide deflection angle range centered on a deflection reference angle direction, is incident on the eye to be examined through the two ellipsoidal concave mirrors (curved mirrors), the deflection reference angle direction for the left eye differs from the deflection reference angle direction for the right eye. Thus, in case of switching the target to be photographed from the left eye to the right eye or from the right eye to the left eye, it is desirable to change the orientation of the ellipsoidal concave mirror(s) facing the eye to be examined, which is to be photographed. This allows to minimize the range of motion of the optical system due to the change in orientation of the ellipsoidal concave mirror(s).

Therefore, the ophthalmic apparatus according to the embodiments is configured with two ellipsoidal concave mirrors as objective optical system that can be rotated around a predetermined rotation axis, and is provided with a photographic optical system for the left eye and a photographic optical system for the right eye. Here, the illumination optical system may be common to the left and right eyes, or the illumination optical system may be provided with an illumination optical system for the left eye and an illumination optical system for the right eye. Further, the ophthalmic apparatus includes an alignment optical system for left eye, and an alignment optical system for right eye. The alignment optical system for left eye is used for performing position matching of the optical system of apparatus with respect to the left eye at the time of photography of the left eye. The alignment optical system for right eye is used for performing position matching of the optical system of apparatus with respect to the right eye at the time of photography of the right eye.

In other words, the ophthalmic apparatus includes a single objective optical system provided in common for the left eye and the right eye, the illumination optical system, and the photographic optical system. At least the photographic optical system includes the photographic optical system for left eye and the photographic optical system for right eye. Further, the ophthalmic apparatus includes the alignment optical system for left eye, and the alignment optical system for right eye.

Hereinafter, the ophthalmic apparatus according to the embodiments will be described specifically.

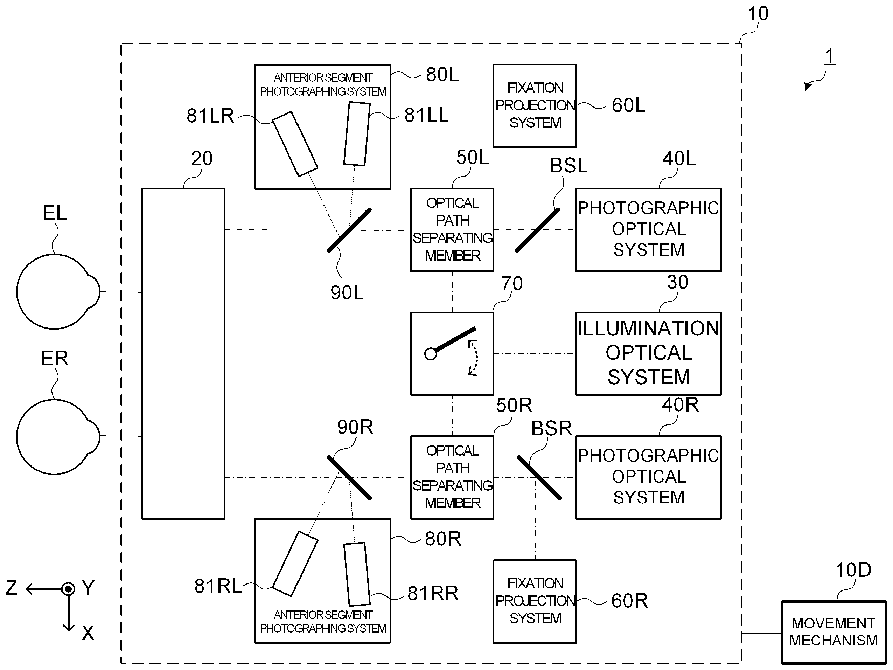

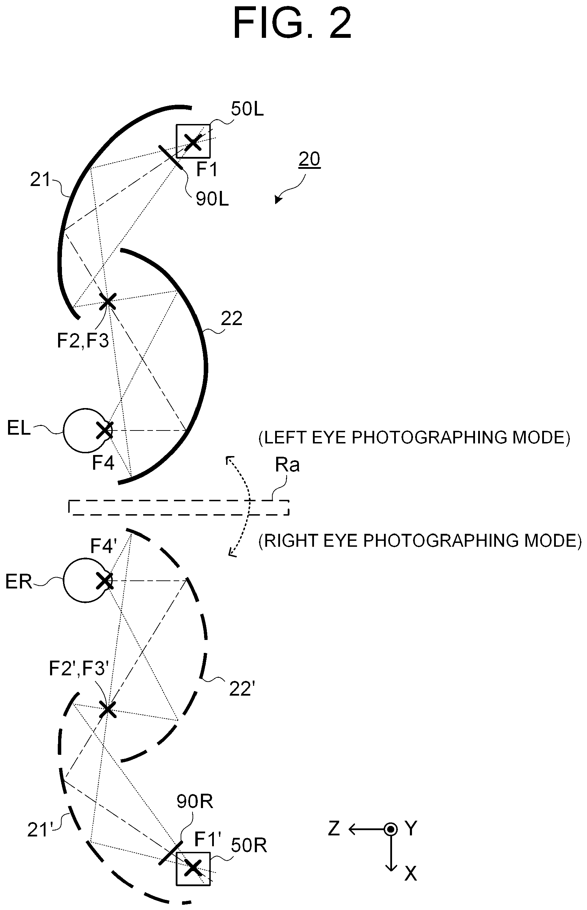

FIGs. 1 to 13 show examples of a configuration of an optical system of the ophthalmic apparatus according to the embodiments. FIG. 1 is a functional block diagram illustrating an example of a configuration of an optical system of the ophthalmic apparatus according to the embodiments. FIGs. 2 to 13 represent examples of the configuration of an optical system 10 shown in FIG. 1. In FIGs. 2 to 13, like parts are designated by like reference numerals as in FIG. 1 and repetitious description of such parts may not be provided.

An ophthalmic apparatus 1 according to the embodiments includes an optical system 10 and a movement mechanism 10D. The optical system 10 is configured to scan a fundus of a left eye EL or a fundus of a right eye ER of an examinee with slit-shaped illumination light, and to sequentially receive returning light from the fundus (fundus of the left eye EL or the right eye ER). The movement mechanism 10D relatively moves the optical system 10 relative to the left eye EL or the right eye ER. The movement mechanism 10D is configured to relatively move the optical system 10 relative to the left eye EL or the right eye ER, by three-dimensionally moving the optical system 10.

The ophthalmic apparatus 1 can switch a photographing operation in accordance with an operation mode. In a left eye photographing mode, the ophthalmic apparatus 1 performs position matching of the optical system 10 with respect to the left eye EL by moving the optical system 10 relative to the left eye EL using the movement mechanism 10D. After then, using the optical system 10, the ophthalmic apparatus 1 scans the fundus of the left eye EL with the slit-shaped illumination light and sequentially receives the returning light from the fundus. In a right eye photographing mode, the ophthalmic apparatus 1 performs position matching of the optical system 10 with respect to the right eye ER by moving the optical system 10 relative to the right eye ER using the movement mechanism 10D. After then, using the optical system 10, the ophthalmic apparatus 1 scans the fundus of the right eye ER with the slit-shaped illumination light and sequentially receives the returning light from the fundus.

The optical system 10 includes an objective optical system 20, an illumination optical system 30, photographic optical systems 40L and 40R, optical path separating members 50L and 50R, fixation projection systems 60L and 60R, an optical path switching member 70, and anterior segment photographing systems 80L and 80R as alignment optical systems. The anterior segment photographing system 80L as the alignment optical system for left eye includes two anterior segment cameras 81LL and 81LR. The anterior segment photographing system 80R as the alignment optical system for right eye includes two anterior segment cameras 81RL and 81RR. Furthermore, the optical system 10 includes dichroic mirrors 90L and 90R, and beam splitters BSL and BSR.

The objective optical system 20 includes a reflective optical system configured to optically relay a measurement position where the pupil of the eye to be examined can be disposed. In the left eye photographing mode, the objective optical system 20 relays the left eye measurement position, where the pupil (iris) of the left eye EL can be disposed, to the left eye measurement conjugate position that is optically conjugate to said measurement position. In the right eye photographing mode, the objective optical system 20 relays the right eye measurement position, where the pupil (iris) of the right eye ER can be disposed, to the right eye measurement conjugate position that is optically conjugate to said measurement position.

The illumination optical system 30 is configured to sequentially irradiate the slit-shaped illumination light onto the left eye EL and the right eye ER through the objective optical system 20. Specifically, the illumination optical system 30 is configured to sequentially illuminate a predetermined irradiated region on the fundus of the left eye EL while deflecting the slit-shaped illumination light in the left eye photographing mode. In addition, the illumination optical system 30 is configured to sequentially illuminate a predetermined irradiated region on the fundus of the right eye ER while deflecting the slit-shaped illumination light in the right eye photographing mode.

The photographic optical system 40L is configured to sequentially receive the returning light of the illumination light from the predetermined irradiated region on the fundus of the left eye EL in the left eye photographing mode. The photographic optical system 40R is configured to sequentially receive the returning light of the illumination light from the predetermined irradiated region on the fundus of the right eye ER in the right eye photographing mode.

The optical path separating member 50L is disposed at the left eye measurement conjugate position described above, and is configured to separate an optical path of the illumination light from the illumination optical system 30 and an optical path of the returning light of the illumination light from the fundus of the left eye EL. The optical path separating member 50R is disposed at the right eye measurement conjugate position described above, and is configured to separate an optical path of the illumination light from the illumination optical system 30 and an optical path of the returning light of the illumination light from the fundus of the right eye ER.

The fixation projection system 60L is configured to project a fixation luminous flux onto the fundus of the left eye EL in the left eye photographing mode. The fixation projection system 60R is configured to project a fixation luminous flux onto the fundus of the right eye ER in the right eye photographing mode.

The optical path switching member 70 is configured to guide the slit-shaped illumination light generated by the illumination optical system 30 and deflected by a deflecting member (optical scanner), which is not shown, to the optical path separating member 50L or the optical path separating member 50R. The optical path switching member 70 guides the slit-shaped illumination light from the illumination optical system 30 to the optical path separating member 50L in the left eye photographing mode. In addition, the optical path switching member 70 guides the slit-shaped illumination light from the illumination optical system 30 to the optical path separating member 50R in the right eye photographing mode.

The two anterior segment cameras 81LL and 81LR in the anterior segment photographing system 80L are disposed so as to view the left eye measurement conjugate position described above, and, in the left eye photographing mode, is configured to substantially simultaneously photograph the anterior segment of the left eye EL from positions away from the optical axis. The two anterior segment cameras 81RL and 81RR in the anterior segment photographing system 80R are disposed so as to view the right eye measurement conjugate position described above, and in the right eye photographing mode, is configured to substantially simultaneously photograph the anterior segment of the right eye ER from positions away from the optical axis.

The dichroic mirror 90L is disposed between the objective optical system 20 and the optical path separating member 50L, and is configured to guide at least part of the light from the left eye EL to the anterior segment photographing system 80L. The light from the left eye EL may be the returning light of the illumination light from the left eye EL or the returning light of the illumination light from the left eye EL illuminated by the anterior segment illumination light source that is not shown. The dichroic mirror 90R is disposed between the objective optical system 20 and the optical path separating member 50R, and is configured to guide at least part of the light from the right eye ER to the anterior segment photographing system 80R. The light from the right eye ER may be the returning light of the illumination light from the right eye ER or the returning light of the illumination light from the right eye ER illuminated by the anterior segment illumination light source that is not shown.

The beam splitter BSL is disposed between the optical path separating member 50L and the photographic optical system 40L, and is configured to reflect the fixation luminous flux from the fixation projection system 60L toward the optical path separating member 50L. The beam splitter BSR is disposed between the optical path separating member 50R and the photographic optical system 40R, and is configured to reflect the fixation luminous flux from the fixation projection system 60R toward the optical path separating member 50R.

Hereinafter, each optical system that makes up the optical system 10 in FIG. 1 will be described specifically.

### (Objective optical system 20)

FIG. 2 shows a diagram for explaining a configuration of the objective optical system 20 shown in FIG. 1. In FIG. 2, a state of objective optical system 20 in the left eye photographing mode is schematically represented by a solid line, and a state of objective optical system 20 in the right eye photographing mode is schematically represented by a broken line. In FIG. 2, like reference numerals designate like parts as in FIG. 1. The same description may not be repeated.

The objective optical system 20 includes a first ellipsoidal concave mirror and a second ellipsoidal concave mirror, and is configured to be capable of rotating the first ellipsoidal concave mirror and the second ellipsoidal concave mirror around a predetermined rotation axis Ra using a rotation mechanism not shown. The rotation axis Ra is an axis in the Z direction passing through a position of the center of gravity of the total mass of the first ellipsoidal concave mirror and the second ellipsoidal concave mirror. This allows a radius of rotation to be shortened while taking weight balance into consideration. In some embodiments, the rotation axis Ra is disposed to pass through a midpoint between the left eye measurement position and the right eye measurement position. In this case, a sliding movement of the optical system 10 described below due to rotation can be made unnecessary. It should be noted that when the left eye measurement position and the right eye measurement position are the same, the rotation axis Ra is disposed to pass through the left eye measurement position and the right eye measurement position.

In FIG. 2, for convenience of explanation, the first ellipsoidal concave mirror and the second ellipsoidal concave mirror are assumed to be denoted as the first ellipsoidal concave mirror 21 and the second ellipsoidal concave mirror 22 in the left eye photographing mode, and to be denoted as the first ellipsoidal concave mirror 21' and the second ellipsoidal concave mirror 22', respectively, in the right eye photographing mode.

A reflective surface of the first ellipsoidal concave mirror 21 (21') is a concave-shaped elliptical surface. The first ellipsoidal concave mirror 21 (21') is an example of the curved mirror or the concave mirror. The first ellipsoidal concave mirror 21 (21') has two optically conjugate focal points (first focal point F1 and second focal point F2, or first focal point F1' and second focal point F2'). The first focal point F1 is a secondary pupil conjugate point of the left eye EL (secondary left eye measurement conjugate position), and the first focal point F1' is a secondary pupil conjugate point of the right eye ER (secondary right eye measurement conjugate position). The second focal point F2 is a primary pupil conjugate point of the left eye EL (primary left eye measurement conjugate position) and the second focal point F2' is a primary pupil conjugate point of the right eye ER (primary right eye measurement conjugate position).

A reflective surface of the second ellipsoidal concave mirror 22 (22') is a concave-shaped elliptical surface. The second ellipsoidal concave mirror 22 (22') is an example of the curved mirror or the concave mirror. The second ellipsoidal concave mirror 22 (22') has two optically conjugate focal points (first focal point F3 and second focal point F4, or first focal point F3' and second focal point F4'). The first focal point F3 is a primary pupil conjugate point of the left eye EL (primary left eye measurement conjugate position) and the first focal point F3' is a primary pupil conjugate point of the right eye ER (primary right eye measurement conjugate position).

The first ellipsoidal concave mirror 21 (21') can be disposed so that the first focal point F2 (F2') coincides with the first focal point F3 (F3') of the second ellipsoidal concave mirror 22 (22') or in the vicinity of the first focal point F3 (F3'). In some embodiments, the first ellipsoidal concave mirror 21 (21') is disposed so that the second focal point F2 (F2') coincides with a position conjugate optically to the first focal point F3 (F3') (conjugate position of the first focal point F3 (F3')) of the second ellipsoidal concave mirror 22 (22') or in the vicinity of the position conjugate optically to the first focal point F3 (F3').

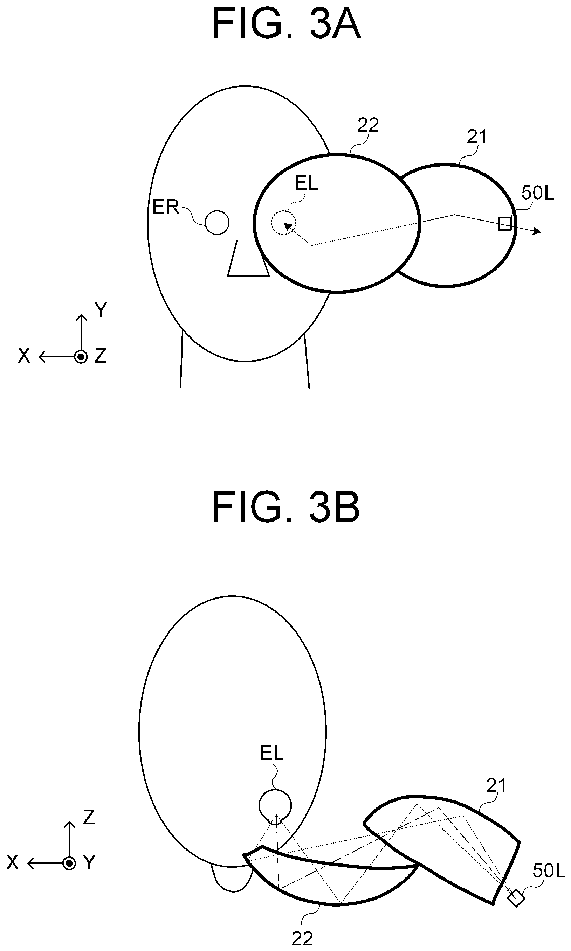

FIG. 3A is a schematic diagram of the state of the objective optical system 20 in the left eye photographing mode when viewed from a front of the examinee. FIG. 3B is a schematic diagram of the state of the objective optical system 20 in the left eye photographing mode when viewed from above the examinee.

In the left eye photographing mode, the first ellipsoidal concave mirror and the second ellipsoidal concave mirror, which are rotated around the rotation axis Ra, are in a first rotational state. In this case, the two optically conjugate focal points (first focal point F1, second focal point F2) of the first ellipsoidal concave mirror 21 are arranged as shown in FIG. 2. That is, the first ellipsoidal concave mirror 21 and the second ellipsoidal concave mirror 22 are configured to relay the left eye measurement position (F4) to the left eye measurement conjugate position (F1). The optical path separating member 50L is disposed at the first focal point F1 of the first ellipsoidal concave mirror 21 or in the vicinity of the first focal point F1. In addition, the dichroic mirror 90L is configured to reflect at least part of the light from the left eye EL reflected by the first ellipsoidal concave mirror 21 toward -Y direction to guide to the anterior segment photographing system 80L.

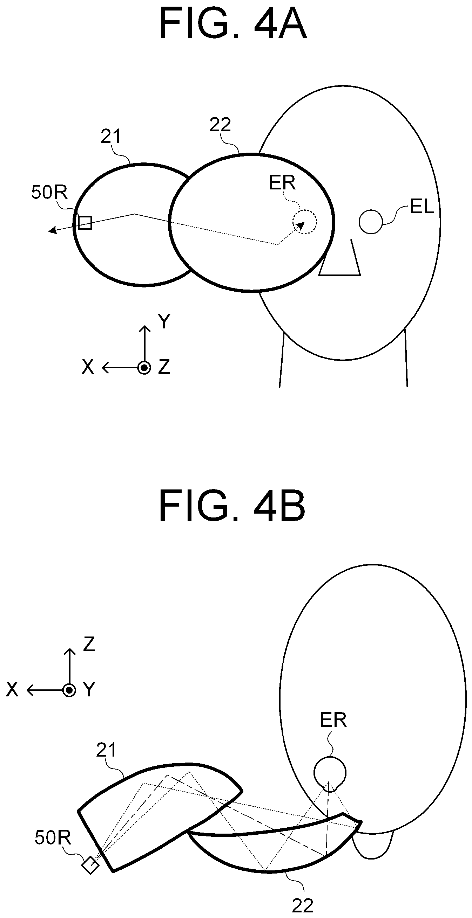

FIG. 4A is a schematic diagram of the state of the objective optical system 20 in the right eye photographing mode when viewed from a front of the examinee. FIG. 4B is a schematic diagram of the state of the objective optical system 20 in the right eye photographing mode when viewed from above the examinee.

In the right eye photographing mode, the first ellipsoidal concave mirror and the second ellipsoidal concave mirror, which are rotated around the rotation axis Ra, are in a second rotational state. For example, the second rotational state is a state where the first ellipsoidal concave mirror and the second ellipsoidal concave mirror are rotated 180 degrees around the rotation axis Ra from the first rotational state. In this case, the two optically conjugate focal points (first focal point F1', second focal point F2') of the first ellipsoidal concave mirror 21' are arranged as shown in FIG. 2. That is, the first ellipsoidal concave mirror 21' and the second ellipsoidal concave mirror 22' are configured to relay the right eye measurement position (F4') to the right eye measurement conjugate position (F1'). The optical path separating member 50R is disposed at the first focal point F1' of the first ellipsoidal concave mirror 21' or in the vicinity of the first focal point F1'. In addition, the dichroic mirror 90R is configured to reflect at least part of the light from the right eye ER reflected by the first ellipsoidal concave mirror 21' toward -Y direction to guide to the anterior segment photographing system 80R.

The ophthalmic apparatus 1 can include an objective system movement mechanism including the rotation mechanism described above, in addition to the movement mechanism for performing position matching of the optical system of apparatus with respect to the eye to be examined. The objective system movement mechanism includes a slide mechanism that slides and moves the optical system, which is obtained by excluding the objective optical system 20 from the optical system 10, in conjunction with the rotation by the rotation mechanism described above.

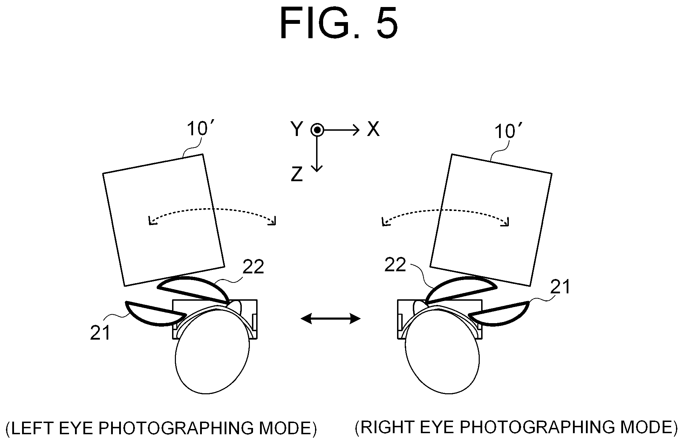

FIG. 5 shows a diagram for explaining a sliding motion of the optical system 10 when viewed from the top of the examinee in case of switching between the left eye photographing mode and the right eye photographing mode.

The slide mechanism described above moves the optical system 10', which is obtained by excluding the objective optical system 20 from the optical system 10, in a direction intersecting the rotation axis Ra (see FIG. 2), in conjunction with the rotation of the first ellipsoidal concave mirror 21 and the second ellipsoidal concave mirror 22 using the rotation mechanism. The optical system 10', which is moved by the slide mechanism, includes illumination optical system 30, the photographic optical systems 40L and 40R, the optical path separating members 50L and 50R, the fixation projection systems 60L and 60R, the optical path switching member 70, the anterior segment photographing systems 80L and 80R, the dichroic mirrors 90L and 90R, and the beam splitters BSL and BSR .

The slide mechanism according to the embodiments can move the optical system 10' along an arc-shaped path centered on a predetermined vertical (Y direction) rotation axis. Examples of the vertical rotation axis include a rotation axis in the Y direction passing through the midpoint between the left eye measurement position and the right eye measurement position, a rotation axis in the Y direction passing through the pupil of the left eye EL disposed at the left eye measurement position, and a rotation axis in the Y direction passing through the pupil of the right eye ER disposed at the right eye measurement position. For example, when switching from the left eye photographing mode to the right eye photographing mode, the slide mechanism moves the optical system 10' along the arc-shaped path centered on the rotation axis in the Y direction passing through the pupil of the left eye EL disposed at the left eye measurement position. For example, when switching from the right eye photographing mode to the left eye photographing mode, the slide mechanism moves the optical system 10' along the arc-shaped path centered on the rotation axis in the Y direction passing through the pupil of the right eye ER disposed at the right eye measurement position.

In some embodiments, the slide mechanism moves the optical system 10' in a straight line direction intersecting the rotation axis Ra, in conjunction with the rotation of the first ellipsoidal concave mirror 21 and the second ellipsoidal concave mirror 22 using the rotation mechanism.

Through such a sliding movement, when switching from the left eye photographing mode to the right eye photographing mode, or switching from the right eye photographing mode to the left eye photographing mode, contact of the first ellipsoidal concave mirror 21 and the second ellipsoidal concave mirror 22 with a nose of the examinee can be avoided.

### (Optical systems other than objective optical system 20)

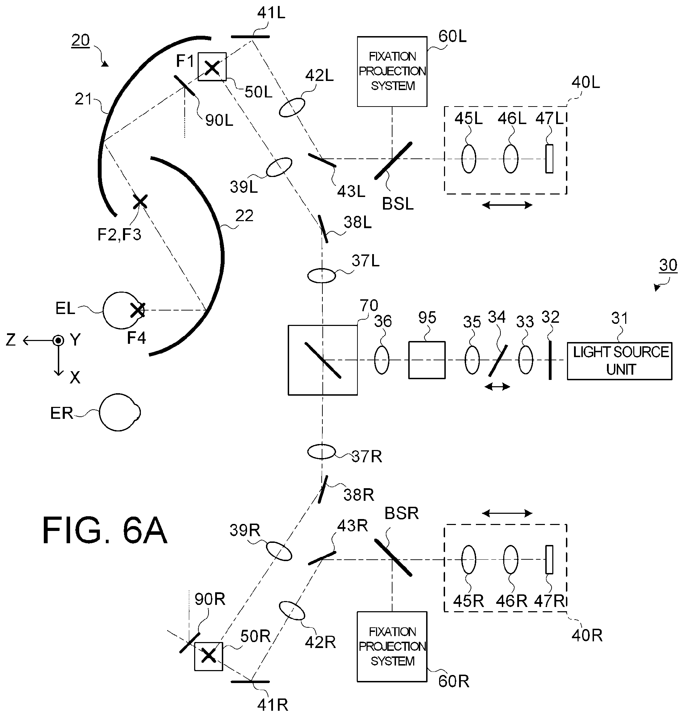

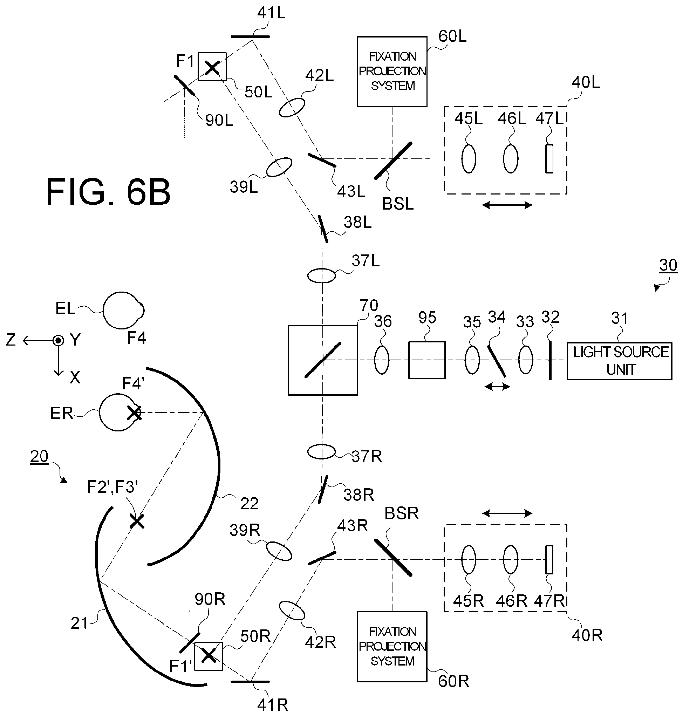

FIG. 6A and FIG. 6B show examples of the configuration of the optical system 10 in FIG. 1. FIG. 6A represents an example of the configuration of the optical system 10 in the left eye photographing mode. FIG. 6B represents an example of the configuration of the optical system 10 in the right eye photographing mode. In FIG. 6A and FIG. 6B, like parts are designated by like reference numerals as in FIG. 1 or FIG. 2 and repetitious description of such parts may not be provided.

### <Illumination optical system 30>

The illumination optical system 30 includes a light source unit 31, an iris aperture 32, a relay lens 33, a slit 34, a relay lens 35, an optical scanner 95, and a relay lens 36.

The light source unit 31 outputs light in the wavelength range including the visible region or the infrared region.

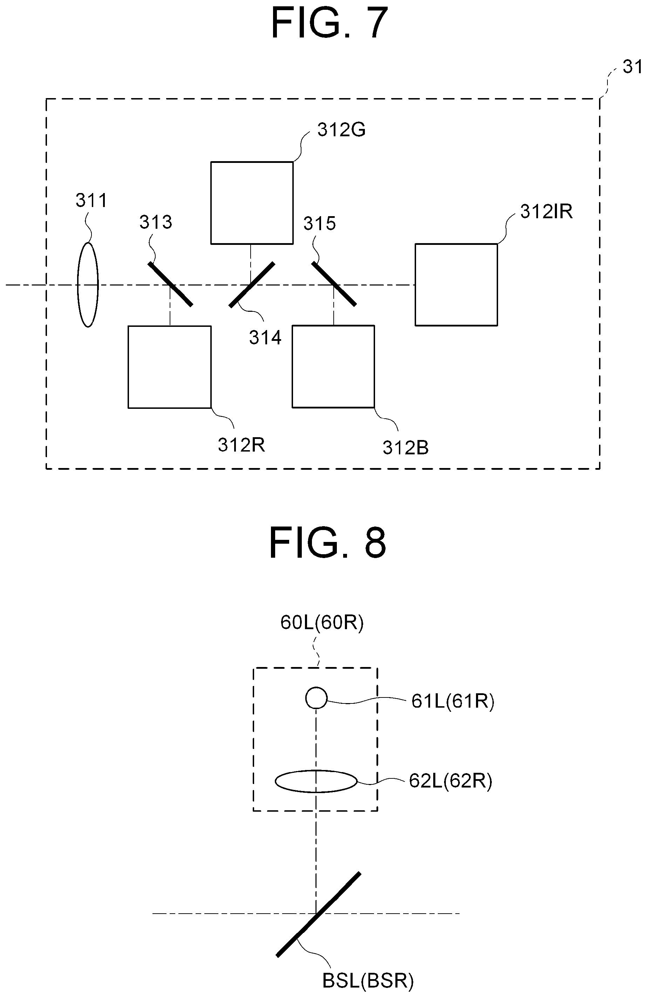

FIG. 7 represents an example of the configuration of the light source unit 31 in FIG. 6A or FIG. 6B.

The light source unit 31 includes a projection lens 311, visible light sources 312R, 312G, and 312B, an infrared light source 312IR, and dichroic mirrors 313, 314, and 315. The visible light source 312R generates light in the wavelength range of the red (R) component. The visible light source 312G generates light in the wavelength range of the green (G) component. The visible light source 312B generates light in the wavelength range of the blue (B) component. The infrared light source 312IR generates light in the wavelength range of near-infrared light.

Each of such visible light sources 312R, 312G, 312B, and infrared light source 312IR includes an LED (Light Emitting Diode), an LD (Laser Diode), for example.

Between the projection lens 311 and the infrared light source 312IR, the dichroic mirrors 313, 314, and 315 are disposed.

The dichroic mirror 313 reflects light in the wavelength range emitted by the visible light source 312R toward the projection lens 311, and transmits light in the wavelength range emitted by the visible light sources 312G and 312B, and the infrared light source 312IR to guide to the projection lens 311.

The dichroic mirror 314 reflects light in the wavelength range emitted by the visible light source 312G toward the dichroic mirror 313, and transmits light in the wavelength range emitted by the visible light source 312B and the infrared light source 312IR to guide to the dichroic mirror 313.

The dichroic mirror 315 reflects light in the wavelength range emitted by the visible light source 312B toward the dichroic mirror 314, and transmits light in the wavelength range emitted by the infrared light source 312IR to guide to the dichroic mirror 314.

By setting the visible light sources 312R, 312G, and 312B to ON and the infrared light source 312IR to OFF, the light source unit 31 can emit white light obtained by synthesizing light from the visible light sources 312R, 312G, and 312B. By setting the visible light sources 312R, 312G, and 312B to OFF and the infrared light source 312IR to ON, the light source unit 31 can emit infrared light from the infrared light source 312IR.

In some embodiments, in a state where the infrared light source 312IR is set to ON or OFF, at least one of the visible light sources 312R, 312G, and 312B is set to ON and at least one of the visible light sources 312R, 312G, and 312B is set to OFF, and the light source unit 31 can emit synthetic light of light from the visible light sources 312R, 312G, and 312B. In this case, the light quantity of the visible light source set to ON among the visible light sources 312R, 312G, and 312B can be changed arbitrarily.

In the iris aperture 32, one or more apertures are formed at position(s) eccentric to an optical axis of the illumination optical system 30. In the present embodiment, a single aperture is formed in the iris aperture. The iris aperture 32 (specifically, its aperture(s)) can be disposed at an iris (pupil) conjugate position. Here, the iris conjugate position is a position conjugate optically to the iris (pupil) of the eye to be examined, which is to be photographed, or in the vicinity of the position conjugate optically to the iris (pupil) of the eye to be examined. In other words, the iris aperture 32 can be disposed at the iris conjugate position of the left eye EL or the right eye ER. The iris aperture 32 functions as an illumination diaphragm. In other words, the aperture formed in the iris aperture 32 defines an incident position (incident shape) of the illumination light on the iris of the eye to be examined, which is to be photographed.

In some embodiments, a relative position between the light source unit 31 and the aperture formed in the iris aperture 32 is configured to be changeable. Thereby, the light amount distribution of the light passing through the aperture(s) formed in the iris aperture 32 can be changed.

In the slit 34, one or more apertures (openings) are formed. In the present embodiment, a single aperture is formed in the slit 34. The aperture formed in the slit 34 is formed so that its longitudinal direction coincides with the major axis direction of the first ellipsoidal concave mirror 21 (the straight line direction connecting the first focal point F1 and the second focal point F2). The slit 34 (specifically, its aperture(s)) can be disposed at a fundus conjugate position. Here, the fundus conjugate position is a position conjugate optically to the fundus of the eye to be examined, which is to be photographed, or in the vicinity of the position conjugate optically to the fundus of the eye to be examined. In other words, the slit 34 can be disposed at the fundus conjugate position of the left eye EL or the right eye ER. The aperture(s) formed in the slit 34 defines a shape of an illuminated region (irradiated pattern shape) on the fundus of the eye to be examined, which is to be photographed. The illumination light emitted from the light source unit 31 passes through the slit 34, is projected onto the reflective surface of the first ellipsoidal concave mirror 21 so that the longitudinal direction of the slit-shaped illumination light roughly coincides with the major axis direction of the first ellipsoidal concave mirror 21, and is guided as slit-shaped illumination light to the fundus of the eye to be examined, which is to be photographed.

The slit 34 is movable in the optical axis direction of the illumination optical system 30 using a movement mechanism (not show) (specifically, movement mechanism 34D described below). This allows to move the position of the slit 34 in accordance with the state (specifically, the dioptric power (refractive power) or the shape of the fundus (fundus curvature)) of the eye to be examined, which is to be photographed.

For example, the storage unit 102 stores first control information. In the first control information, the positions of the slit 34 on the optical axis of the illumination optical system 30 are associated with the dioptric powers in advance. The main controller 101 identifies the position of the slit 34 corresponding to the dioptric power by referring to the first control information, and controls the movement mechanism 34D so that the slit 34 is disposed at the identified position.

Here, as the slit 34 moves, the light quantity distribution of the light passing through the aperture formed in the slit 34 changes. In this case, the main controller 101 can control the movement mechanism (not shown) that moves the light source unit 31 to change at least one of the position of the light source included in the light source unit 31 or the orientation of the light source included in the light source unit 31.

In some embodiments, the slit 34 is configured so that at least one of the position of the aperture or the shape of the aperture can be changed in accordance with the state of the eye to be examined, which is to be photographed, without being moved in the optical axis direction. The function of the slit 34 with these functions is, for example, realized by a liquid crystal shutter.

The slit 34 according to the embodiments is configured to be capable of being disposed on the optical axis of the illumination optical system 30 with a different intersection angle (inclination angle) relative to the optical axis of the illumination optical system 30, in accordance with the eye to be examined, which is to be photographed (see FIG. 6A and FIG. 6B).

Specifically, the slit 34 is disposed to be slanted at an intersection angle corresponding to the major axis direction (orientation of the line connecting the two focal points) of the first ellipsoidal concave mirror 21, into which the slit-shaped illumination light enters, on the optical axis of the illumination optical system 30. For example, in the left eye photographing mode, the slit 34 is disposed on the optical axis of the illumination optical system 30 so that the major axis direction (the direction of the line connecting the first focal point F1 and the second focal point F2) of the first ellipsoidal concave mirror 21 shown in FIG. 6A and the exit plane of the slit 34 are optically approximately parallel. In addition, for example, in the right eye photographing mode, the slit 34 is disposed on the optical axis of the illumination optical system 30 so that the major axis direction (the direction of the line connecting the first focal point F1' and the second focal point F2') of the first ellipsoidal concave mirror 21 shown in FIG. 6B and the exit plane of the slit 34 are optically approximately parallel. Here, the term "optically approximate parallel" means not only a state of being approximately parallel on the optical axis (optical path) extending in a straight line in a real space, but also a state substantially equivalent to a state of being approximately parallel on a virtual optical axis from which the reflecting member, etc. is removed from the optical axis deflected by the reflecting member, etc.

The optical scanner 95 deflects the slit-shaped illumination light generated by irradiating the illumination light from the light source unit 31 onto the slit 34. The optical scanner 95 (specifically, its deflection surface) can be disposed at the iris conjugate position. The iris conjugate position is a position conjugate optically to the iris (pupil) of the eye to be examined, which is to be photographed, or in the vicinity of the position conjugate optically to the iris of the eye to be examined. The optical scanner 95 is a uniaxial optical scanner that deflects an orientation of the deflection surface centered on a predetermined deflection reference angle direction. The optical scanner 95 deflects the slit-shaped illumination light one-dimensionally. The optical scanner 95 deflects the illumination light in a direction intersecting (specifically, perpendicular to) the longitudinal direction of the slit image formed by the slit-shaped illumination light projected onto the fundus of the eye to be examined, which is to be photographed. Thereby, the slit image moves in the direction intersecting the longitudinal direction of the slit image (scan direction).

The optical scanner 95 includes, for example, a galvanometer scanner, a MEMS (Micro Electro Mechanical System) scanner, a polygon mirror, or a resonant scanner. For example, the optical scanner 95 includes the galvanometer scanner that deflects the illumination light within a predetermined deflection angular range with reference to a predetermined deflection reference angle direction.

In some embodiments, the optical scanner 95 is a biaxial optical scanner that two-dimensionally deflects the slit-shaped illumination light. For example, the optical scanner 95 includes a first scanner and a second scanner. The first scanner deflects the illumination light so as to move the irradiated region on the fundus of the eye to be examined, which is to be photographed, in a horizontal direction perpendicular to the optical axis of the illumination optical system 30. The second scanner deflects the illumination light deflected by the first scanner so as to move the irradiated region on the fundus in a vertical direction perpendicular to the optical axis of the illumination optical system 30.

In the optical system with the configuration described above, the illumination light in the visible or infrared region emitted from the light source unit 31 is irradiated onto the iris aperture 32, passes through the aperture formed in the iris aperture 32, is transmitted through the relay lens 33, and is guided to the slit 34.

In the left eye photographing mode, the slit 34 is disposed so that the major axis direction (the direction of the line connecting the first focal point F1 and the second focal point F2) of the first ellipsoidal concave mirror 21 and the exit plane of the slit 34 are optically approximately parallel, as shown in FIG. 6A. In the right eye photographing mode, the slit 34 is disposed so that the major axis direction (the direction of the line connecting the first focal point F1' and the second focal point F2') of the first ellipsoidal concave mirror 21 and the exit plane of the slit 34 are optically approximately parallel, as shown in FIG. 6B. The slit-shaped illumination light passing through the aperture formed in the slit 34 is transmitted through the relay lens 35, is deflected by the optical scanner 95, is transmitted through the relay lens 36, and is guided to the optical path switching member 70.

In some embodiments, the illumination optical system 30 includes a projector with a light source, and the projector emits the slit-shaped illumination light. In this case, the projector is provided instead of the light source unit 31, the iris aperture 32, the relay lens 33, and the slit 34 in FIG. 6A and FIG. 6B. Examples of the projector include an LCD (Liquid Crystal Display) type projector using a transmissive LCD panel, an LCOS (Liquid Crystal On Silicon) type projector using a reflective LCD panel, a DLP (Digital Light Processing) (registered trademark) type projector using a DMD (Digital Mirror Device).

The optical path switching member 70 switches the optical path of the slit-shaped illumination light deflected by the optical scanner 95 in accordance with the eye to be examined, which is to be photographed. Specifically, the optical path switching member 70 guides the slit-shaped illumination light to the left eye measurement conjugate position (first focal point F1 in FIG. 6A) or the right eye measurement conjugate position (first focal point F1' in FIG. 6B), which are described above, in accordance with the photographing mode.

The relay lens 37L, the reflecting member 38L, and the relay lens 39L are disposed between the optical path switching member 70 and the left eye measurement conjugate position, where the optical path separating member 50L is disposed. The relay lens 37R, the reflecting member 38R, and the relay lens 39R are disposed between the optical path switching member 70 and the right eye measurement conjugate position, where the optical path separating member 50R is disposed.

When the left eye photographing mode is set, the optical path switching member 70 guides the slit-shaped illumination light transmitted through the relay lens 36 to the relay lens 37L. When the right eye photographing mode is set, the optical path switching member 70 guides the slit-shaped illumination light transmitted through the relay lens 36 to the relay lens 37R. The function of such an optical path switching member 70 can be realized using a known optical path switching member such as a flip mirror.

In some embodiments, the illumination optical system 30 further includes an optical path switching member 70, the relay lenses 37L and 37R, the reflecting members 38L and 38R, and relay lenses 39L and 39R.

By adjusting the orientation of the optical axis of the illumination optical system 30 with the optical path switching member 70 and the reflecting members 38L and 38R, the size of the optical system 10 in the X and Y directions can be reduced. For example, by changing the orientation of the optical axis in the +X direction by the reflecting member 38L and in the -X direction by the reflecting member 38R, the size of the optical system 10 in the X direction can be reduced.

### <Photographic optical system 40L, 40R>

The photographic optical system 40L includes a relay lens 45L, an imaging lens 46L, and an image sensor 47L. The photographic optical system 40L is configured to be capable of being moved in the optical axis direction in an integrated manner. Thereby, the light receiving surface of the image sensor 47L can be disposed at the fundus conjugate position, which is a position conjugate optically to the fundus of the left eye EL or in the vicinity of the position conjugate optically to the fundus of the left eye EL. As a result, this allows to adapt the state of the left eye EL and to image the returning light from the left eye EL on the light receiving surface of the image sensor 47L. In some embodiments, the photographic optical system 40L includes a focusing lens, and is configured to allow the light receiving surface of the image sensor 47L to be disposed at the fundus conjugate position described above by moving the focusing lens in the optical axis direction.

The returning light from the left eye EL is scattered light (reflected light) of the illumination light incident on the left eye EL. In some embodiments, the returning light from the left eye EL includes scattered light (reflected light) of the illumination light incident on the left eye EL, and fluorescence and its scattered light using the illumination light incident on the left eye EL as excitation light.

The image sensor 47L realizes the function of the two-dimensional image sensor as a pixelated photodetector. The light receiving surface (detecting surface, imaging surface) of the image sensor 47L can be disposed at the fundus conjugate position described above. The image sensor 47L is configured to be capable of setting a light receivable region (light-receptive region) that can be virtually moved at the fundus conjugate position.

For example, the light receiving result(s) acquired by the image sensor 47L is/are captured and read out using a rolling shutter system (method). In some embodiments, the light receiving result(s) acquired by the image sensor 47L is/are captured and read out using a global shutter system in which the light receivable region can be changed or be moved. In some embodiments, the controller described below performs readout control of the light receiving result(s) by controlling the image sensor 47L. In some embodiments, the image sensor 47L can automatically output the light receiving result(s) for a predetermined number of lines, along with information indicating the light receiving position(s).

The image sensor 47L with such a configuration includes a CMOS (Complementary Metal Oxide Semiconductor) image sensor. In this case, the image sensor 47L includes a plurality of pixels (light receiving elements). The pixels includes a plurality of pixel groups arranged in a column direction. Each of the pixel groups includes pixels arranged in a row direction. Specifically, the image sensor 47L includes a plurality of pixels arranged two-dimensionally, a plurality of vertical signal lines, and a horizontal signal line.

In some embodiments, the image sensor 47L is a CCD (Charge Coupled Device) image sensor, for example.

By capturing (reading out) the light receiving result(s) of the returning light using the rolling shutter system for such an image sensor 47L, the image in the light receivable region corresponding to a desired virtual opening shape extending in the row direction is acquired. Such control is disclosed in, for example, U.S. Patent No. 7,831,106, U.S. Patent No. 8,237,835, or the like.

A reflecting member 41L, a relay lens 42L, a reflecting member 43L, and a beam splitter BSL are disposed between the optical path separating member 50L and the photographic optical system 40L. The fixation projection system 60L described below is disposed in a reflection direction of the beam splitter BSL. In some embodiments, the photographic optical system 40L includes the reflecting member 41L, the relay lens 42L, the reflecting member 43L, and the beam splitter BSL.

The returning light of the illumination light from the left eye EL, which is separated by the optical path separating member 50L, is reflected by the reflecting member 41L, is transmitted through the relay lens 42L, is reflected by the reflecting member 43L, is transmitted through the beam splitter BSL, and is guided to the photographic optical system 40L. The returning light guided to the photographic optical system 40L is transmitted through the relay lens 45L, and images on the light receiving surface on the image sensor 47L by the imaging lens 46L.

On the other hand, the photographic optical system 40R has the same configuration as the photographic optical system 40L. In other words, the photographic optical system 40R includes a relay lens 45R, an imaging lens 46R, and an image sensor 47R. The photographic optical system 40R is configured to be capable of being moved in the optical axis direction in an integrated manner. Thereby, the light receiving surface of the image sensor 47R can be disposed at the fundus conjugate position, which is a position conjugate optically to the fundus of the right eye ER or in the vicinity of the position conjugate optically to the fundus of the right eye ER. As a result, this allows to adapt the state of the right eye ER and to image the returning light from the right eye ER on the light receiving surface of the image sensor 47R. In some embodiments, the photographic optical system 40R includes a focusing lens, and is configured to allow the light receiving surface of the image sensor 47R to be disposed at the fundus conjugate position described above by moving the focusing lens in the optical axis direction.

The returning light from the right eye ER is scattered light (reflected light) of the illumination light incident on the right eye ER. In some embodiments, the returning light from the right eye ER includes scattered light (reflected light) of the illumination light incident on the right eye ER, and fluorescence and its scattered light using the illumination light incident on the right eye ER as excitation light.

The image sensor 47R realizes the function of the two-dimensional image sensor as a pixelated photodetector, in the same way as the image sensor 47L. The light receiving surface (detecting surface, imaging surface) of the image sensor 47R can be disposed at the fundus conjugate position described above. The image sensor 47R is configured to be capable of setting a light receivable region (light-receptive region) that can be virtually moved at the fundus conjugate position.

The light receiving result acquired by the image sensor 47R is also read out using a rolling shutter system, in the same way as the image sensor 47L. In some embodiments, the light receiving result(s) acquired by the image sensor 47R is/are captured and read out using a global shutter system in which the light receivable region can be changed or be moved. In some embodiments, the controller described below performs readout control of the light receiving result(s) by controlling the image sensor 47R. In some embodiments, the image sensor 47R can automatically output the light receiving result(s) for a predetermined number of lines, along with information indicating the light receiving position(s).

The image sensor 47R with this configuration includes a CMOS image sensor, in the same way as the image sensor 47L. In some embodiments, the image sensor 47R is a CCD image sensor, for example.

By capturing (reading out) the light receiving result(s) of the returning light using the rolling shutter system for such an image sensor 47R, the image in the light receivable region corresponding to a desired virtual opening shape extending in the row direction is acquired.

A reflecting member 41R, a relay lens 42R, a reflecting member 43R, and a beam splitter BSR are disposed between the optical path separating member 50R and the photographic optical system 40R. The fixation projection system 60R described below is disposed in a reflection direction of the beam splitter BSR. In some embodiments, the photographic optical system 40R includes the reflecting member 41R, the relay lens 42R, the reflecting member 43R, and the beam splitter BSR.

The returning light of the illumination light from the right eye ER, which is separated by the optical path separating member 50R, is reflected by the reflecting member 41R, is transmitted through the relay lens 42R, is reflected by the reflecting member 43R, is transmitted through the beam splitter BSR, and is guided to the photographic optical system 40R. The returning light guided to the photographic optical system 40R is transmitted through the relay lens 45R, and images on the light receiving surface on the image sensor 47R by the imaging lens 46R.

By adjusting the orientations of the optical axes of the photographic optical systems 40L and 40R with the reflecting members 41L, 43L, 41R, and 43R, the size of the optical system 10 in the X and Y directions can be reduced. For example, by changing the orientation of the optical axis in the +X direction by the reflecting members 41L and 43L and in the -X direction by the reflecting members 41R and 43R, the size of the photographic optical systems 40L and 40R can be reduced. For example, using the reflecting members 41L and 43L, the orientation of the optical axis of the photographic optical system 40L can be adjusted to be approximately coincident with the Z direction. For example, using the reflecting members 41R and 43R, the orientation of the optical axis of the photographic optical system 40R can be adjusted to be approximately coincident with the Z direction.

### <Fixation projection system 60L, 60R>

The fixation projection system 60L is configured to project a fixation luminous flux onto the fundus of the left eye EL in the left eye photographing mode. The fixation projection system 60R is configured to project a fixation luminous flux onto the fundus of the right eye ER in the right eye photographing mode.

The fixation projection system 60L has the same configuration as the fixation projection system 60R.

FIG. 8 represents an example of the configuration of the fixation projection system 60L in FIG. 6A (fixation projection system 60R in FIG. 6B).

The fixation projection system 60L includes a fixation light source 61L and a projection lens 62L. The fixation light source 61L can be disposed at the fundus conjugate position, which is a position conjugate optically to the fundus of the left eye EL or in the vicinity of the position conjugate optically to the fundus of the left eye EL. The fixation luminous flux emitted by fixation light source 61L is transmitted through the projection lens 62L, is reflected by the beam splitter BSL, passes through the reflecting member 43L, the relay lens 42L, and the reflecting member 41L, and is guided to the optical path separating member 50L. The fixation luminous flux guided to the optical path separating member 50L is transmitted through the dichroic mirror 90L, and is projected onto the fundus of the left eye EL through the first ellipsoidal concave mirror 21 and the second ellipsoidal concave mirror 22 (see FIG. 6A).

The fixation projection system 60R includes a fixation light source 61R and a projection lens 62R. The fixation light source 61R can be disposed at the fundus conjugate position, which is a position conjugate optically to the fundus of the right eye ER or in the vicinity of the position conjugate optically to the fundus of the right eye ER. The fixation luminous flux emitted by fixation light source 61R is transmitted through the projection lens 62R, is reflected by the beam splitter BSR, passes through the reflecting member 43R, the relay lens 42R, and the reflecting member 41R, and is guided to the optical path separating member 50R. The fixation luminous flux guided to the optical path separating member 50R is transmitted through the dichroic mirror 90R, and is projected onto the fundus of the right eye ER through the first ellipsoidal concave mirror 21 and the second ellipsoidal concave mirror 22 (see FIG. 6B).

### <Optical path separating member 50L, 50R>

The optical path separating member 50L is disposed at the left eye measurement conjugate position, which is a position substantially conjugate optically to the left eye measurement position, as described above. In addition, the optical path separating member 50R is disposed at the right eye measurement conjugate position, which is a position substantially conjugate optically to the right eye measurement position, as described above.

The optical path separating member 50L has the same configuration as the optical path separating member 50R.

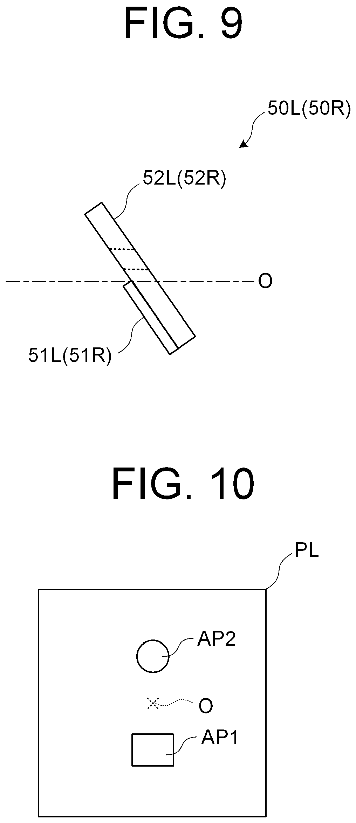

FIG. 9 schematically shows an example of the configuration of the optical path separating member 50L. FIG. 9 schematically represents a cross-sectional structure of the optical path separating member 50L.

The optical path separating member 50L includes a reflecting member 51L as a pupil dividing mirror, and a photographic diaphragm 52L. In the photographic diaphragm 52L, an aperture is formed at a position eccentric from the optical axis O. The reflecting member 51L is provided at a position eccentric from the optical axis O on a surface, which is on the reflective surface side of the first ellipsoidal concave mirror 21, of the photographic diaphragm 52L. In other words, each of the reflecting member 51L and the photographic diaphragm 52L is disposed at the left eye measurement conjugate position.

FIG. 10 schematically represents a conjugate plane at the left eye measurement conjugate position.

The conjugate plane PL is a virtual plane perpendicular to the optical axis at the left eye measurement conjugate position on the optical axis O. In the conjugate plane PL, an image AP1 of the aperture formed in the iris aperture 32 and an image AP2 of the aperture formed in the photographic diaphragm 52L are disposed. In this case, the image of the aperture formed in the photographic diaphragm 52L and the image of the aperture formed in the iris aperture 32 are arranged so as to be non-overlapping.

Therefore, in the optical path separating member 50L, the reflecting member 51L reflects the slit-shaped illumination light from the illumination optical system 30 to guide to the objective optical system 20 (first ellipsoidal concave mirror 21). The photographic diaphragm 52L guides the returning light passing through its aperture to the photographic optical system 40L.

Similarly, the optical path separating member 50R includes a reflecting member 51R as a pupil dividing mirror, and a photographic diaphragm 52R. In the photographic diaphragm 52R, an aperture is formed at a position eccentric from the optical axis. The reflecting member 51R is provided at a position eccentric from the optical axis on a surface, which is on the reflective surface side of the first ellipsoidal concave mirror 21, of the photographic diaphragm 52R. In other words, each of the reflecting member 51R and the photographic diaphragm 52R is disposed at the right eye measurement conjugate position.

In other words, on a conjugate plane at the right eye measurement conjugate position, an image of the aperture formed in the iris aperture 32 and an image of the aperture formed in the photographic diaphragm 52R are disposed, in the same way as in FIG. 10. Therefore, in the optical path separating member 50R, the reflecting member 51R reflects the slit-shaped illumination light from the illumination optical system 30 to guide to the objective optical system 20 (first ellipsoidal concave mirror 21). The photographic diaphragm 52R guides the returning light passing through its aperture to the photographic optical system 40R.

### <Anterior segment photographing system 80L, 80R>

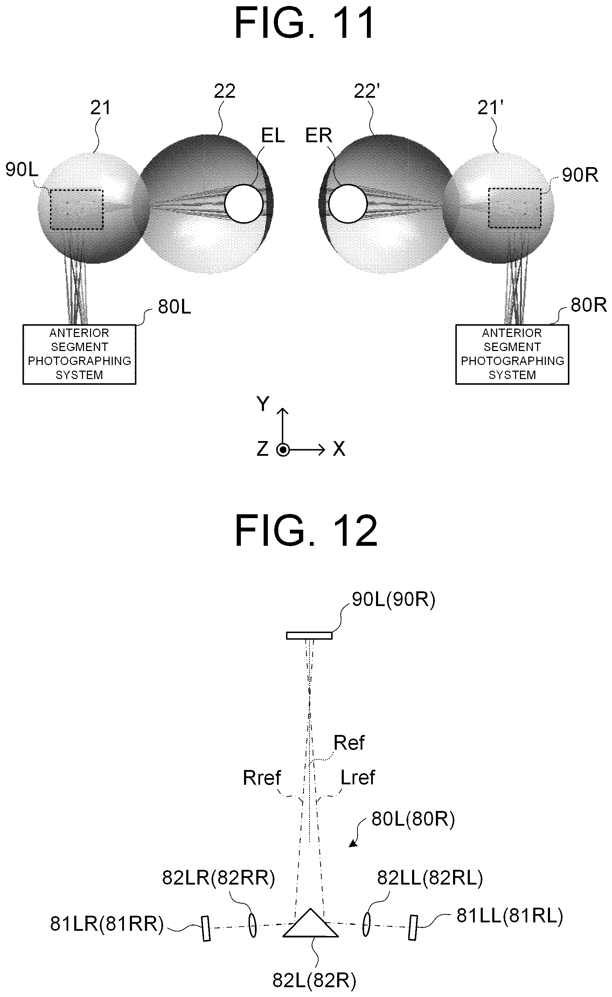

FIG. 11 schematically represents a positional relationship between the objective optical system 20 and the anterior segment photographing systems 80L and 80R when viewed from the reflective surface side of the second ellipsoidal concave mirror 22 (22'). In FIG. 11, parts similar to those in FIG. 1, FIG. 2, FIG. 6A, or FIG. 6B are denoted by the same reference symbols, and description thereof is omitted as appropriate.

The anterior segment photographing system 80L is disposed in the -Y direction, which is a reflection direction of the dichroic mirror 90L, as shown in FIG. 11. The anterior segment photographing system 80R is disposed in the -Y direction, which is a reflection direction of the dichroic mirror 90R, as shown in FIG. 11. Thereby, the size of the optical system 10 at least in the X direction can be reduced.

FIG. 12 schematically shows an example of the configuration of the anterior segment photographing system 80L (80R). In FIG. 12, like reference numerals designate like parts as in FIG. 1 or FIG. 11. The same description may not be repeated.

The anterior segment photographing system 80L includes an optical path deflecting prism 82L and imaging lenses 82LL and 82LR, in addition to the two anterior segment cameras 81LL and 81LR.

The light receiving surface (imaging surface) of each of the anterior segment cameras 81LL and 81LR can be disposed at the fundus conjugate position, which is a position conjugate optically to the fundus of the left eye EL or in the vicinity of the position conjugate optically to the fundus of the left eye EL. For example, the anterior segment cameras 81LL and 81LR are disposed so that angles formed by a left eye measurement conjugate plane, which is perpendicular to the optical axis at the left eye measurement conjugate position, and photographic optical axes Lref, Rref are the same, and that the angles are symmetrical with reference to a normal direction (optical axis direction) of the left eye measurement conjugate plane. In some embodiments, the light receiving surface (imaging plane) is positioned at the fundus conjugate position by moving the anterior segment cameras 81LL and 81LR in the optical axis direction. In some embodiments, two focusing lenses are provided between the dichroic mirror 90L and each of the imaging lenses 82LL and 82LR, and the light receiving surface (imaging surface) can be disposed at the fundus conjugate position by moving each of the two focusing lenses in the optical axis direction.

For example, the photographic reference optical axis Ref of the anterior segment photographing system 80L, which is deflected by the dichroic mirror 90L, passes through the midpoint of a baseline connecting the anterior segment cameras 81LL and 81LR, and is an optical axis perpendicular to the baseline. The photographic optical axis Lref of the anterior segment camera 81LL and the photographic optical axis Rref of the anterior segment camera 81LR are disposed so as to pass through a position optically equivalent to the left eye measurement conjugate position. That is, each of the anterior segment cameras 81LL and 81LR is disposed on the photographic optical axes Lref and Rref, respectively, which intersect the photographic reference optical axis Ref.

In some embodiments, as shown in FIG. 12, the optical path deflecting prism 82L is provided. The optical path deflecting prism 82L is a triangular prism that has a first deflection surface and a second deflection surface that intersect at a predetermined intersection angle and share a ridge line, for example. In this case, the returning light reflected by the dichroic mirror 90L is deflected by the first deflection surface of the optical path deflecting prism 82L toward the imaging lens 82LL, and forms on the light receiving surface of the anterior segment camera 81LL by the imaging lens 82LL. In addition, the returning light reflected by the dichroic mirror 90L is deflected by the second deflection surface of the optical path deflecting prism 82L toward the imaging lens 82LR, and forms on the light receiving surface of the anterior segment camera 81LR by the imaging lens 82LR.

Thereby, the anterior segment cameras 81LL and 81LR can be arranged so as to optically view the left eye measurement conjugate position while avoiding physical interference of the anterior segment cameras 81LL and 81LR.

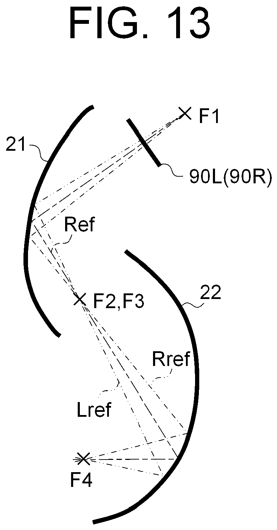

FIG. 13 schematically represents photographic optical axes of the anterior segment cameras 81LL and 81LR in the objective optical system 20. In FIG. 13, parts similar to those in FIG. 1, FIG. 2, FIG. 11, or FIG. 12 are denoted by the same reference symbols, and description thereof is omitted as appropriate.

As shown in FIG. 13, the anterior segment cameras 81LL and 81LR are disposed so as to view the left eye measurement conjugate position (or a position conjugate optically to the left eye measurement conjugate position), which is optically equivalent to the left eye measurement position where the left eye EL is disposed. In other words, the anterior segment cameras 81LL and 81LR can photograph the left eye EL from a position away from the optical axis of the illumination optical system 30 and the photographic optical system 40L.

Similarly, the anterior segment photographing system 80R includes an optical path deflecting prism 82R and imaging lenses 82RL and 82RR, in addition to the two anterior segment cameras 81RL and 81RR (see FIG. 12).

The light receiving surface (imaging surface) of each of the anterior segment cameras 81RL and 81RR can be disposed at the fundus conjugate position, which is a position conjugate optically to the fundus of the right eye ER or in the vicinity of the position conjugate optically to the fundus of the right eye ER. For example, the anterior segment cameras 81RL and 81RR are disposed so that angles formed by a right eye measurement conjugate plane, which is perpendicular to the optical axis at the right eye measurement conjugate position, and the photographic optical axes are the same, and that the angles are symmetrical with reference to a normal direction (optical axis direction) of the right eye measurement conjugate plane. In some embodiments, the light receiving surface (imaging plane) is positioned at the fundus conjugate position by moving the anterior segment cameras 81RL and 81RR in the optical axis direction. In some embodiments, two focusing lenses are provided between the dichroic mirror 90R and each of the imaging lenses 82RL and 82RR, and the light receiving surface (imaging surface) can be disposed at the fundus conjugate position by moving each of the two focusing lenses in the optical axis direction.

For example, the photographic reference optical axis Ref of the anterior segment photographing system 80R, which is deflected by the dichroic mirror 90R, passes through the midpoint of a baseline connecting the anterior segment cameras 81RL and 81RR, and is an optical axis perpendicular to the baseline. The photographic optical axis Lref of the anterior segment camera 81RL and the photographic optical axis Rref of the anterior segment camera 81RR are disposed so as to pass through a position optically equivalent to the right eye measurement conjugate position. That is, each of the anterior segment cameras 81RL and 81RR is disposed on the photographic optical axes Lref and Rref, respectively, which intersect the photographic reference optical axis Ref.

In some embodiments, as shown in FIG. 12, the optical path deflecting prism 82R is provided. The optical path deflecting prism 82R is a triangular prism that has a first deflection surface and a second deflection surface that intersect at a predetermined intersection angle and share a ridge line, for example. In this case, the returning light reflected by the dichroic mirror 90R is deflected by the first deflection surface of the optical path deflecting prism 82R toward the imaging lens 82RL, and forms on the light receiving surface of the anterior segment camera 81RL by the imaging lens 82RL. In addition, the returning light reflected by the dichroic mirror 90R is deflected by the second deflection surface of the optical path deflecting prism 82R toward the imaging lens 82RR, and forms on the light receiving surface of the anterior segment camera 81RR by the imaging lens 82RR.

Thereby, the anterior segment cameras 81RL and 81RR can be arranged so as to optically view the left eye measurement conjugate position while avoiding physical interference of the anterior segment cameras 81RL and 81RR.

Therefore, the anterior segment cameras 81RL and 81RR are disposed so as to view the right eye measurement conjugate position (or a position conjugate optically to the right eye measurement conjugate position), which is optically equivalent to the right eye measurement position where the right eye ER is disposed, in the same way as the anterior segment cameras 81LL and 81LR. In other words, the anterior segment cameras 81RL and 81RR can photograph the right eye ER from a position away from the optical axis of the illumination optical system 30 and the photographic optical system 40R.

In some embodiments, at least one of the first ellipsoidal concave mirror 21 or the second ellipsoidal concave mirror 22 is a convex mirror whose reflective surface is formed in a convex shape (for example, ellipsoidal convex mirror). In some embodiments, at least one of the first ellipsoidal concave mirror 21 or the second ellipsoidal concave mirror 22 is a curved mirror whose reflective surface is a free-form surface.

In addition, the ophthalmic apparatus 1 may be provided with any element or unit, such as a member for supporting the face of the examinee (chin rest, forehead pad, etc.).

The anterior segment photographing system 80L or the anterior segment photographing system 80R is an example of the "photographing unit" according to the embodiments. The optical path separating member 50L or the optical path separating member 50R is an example of the "first optical path separating member (first optical path separator)" according to the embodiments. The dichroic mirror 90L or the dichroic mirror 90R is an example of the "second optical path separating member (second optical path separator)" according to the embodiments. The iris aperture 32 is an example of the "illumination diaphragm" according to the embodiments. The anterior segment photographing system 80L is an example of the "photographing unit for left eye" according to the embodiments. The anterior segment photographing system 80R is an example of the "photographing unit for right eye" according to the embodiments. The photographic optical system 40L is an example of the "photographic optical system for left eye" according to the embodiments. The photographic optical system 40R is an example of the "photographic optical system for right eye" according to the embodiments. The first ellipsoidal concave mirror 21 and the second ellipsoidal concave mirror 22 are an example of the "two concave mirrors" according to the embodiments. The optical path separating member 50L is an example of the "optical path separating member for left eye (optical path separator for left eye)" according to the embodiments. The optical path separating member 50R is an example of the "optical path separating member for right eye (optical path separator for right eye)" according to the embodiments.

### <Control system>

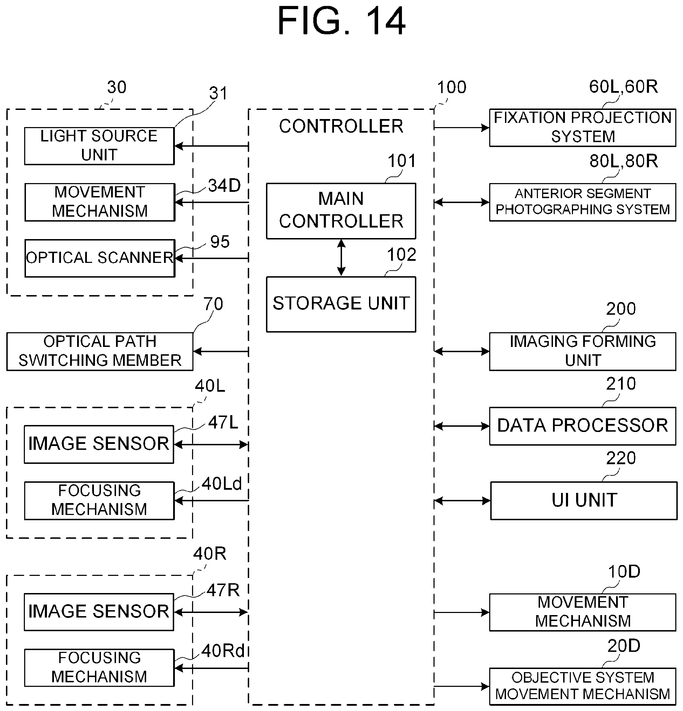

FIG. 14 shows an example of a control system of the ophthalmic apparatus 1 according to the embodiments. In FIG. 14, parts similar to those in FIG. 1 to FIG. 13 are denoted by the same reference symbols, and description thereof is omitted as appropriate.

The control system (processing system) of the ophthalmic apparatus 1 is configured with the controller 100 as a center. The controller 100 controls each part of the ophthalmic apparatus 1.

The controller 100 includes a main controller 101 and a storage unit 102. The functions of the main controller 101 are realized by a processor, for example. The storage unit 102 stores, in advance, computer program(s) for controlling the ophthalmic apparatus 1. The computer programs include program for controlling the illumination optical system, program for controlling the photographic optical system, program for controlling the optical scanner, program for controlling the anterior segment photographing system, program for image forming, program for data processing, and program for user interface. The main controller 101 operates according to such the computer program(s), and thereby the controller 100 performs the control processing.

### (Main controller 101)

The main controller 101 controls each part of the objective optical system 20 (objective system movement mechanism 20D), the illumination optical system 30, the photographic optical systems 40L and 40R, the fixation projection systems 60L and 60R, the optical path switching member 70, the anterior segment photographing systems 80L and 80R, and the movement mechanism 10D. Further, the main controller 101 controls an image forming unit 200, a data processor 210, and a user interface (UI) unit 220.

Examples of the control for the objective optical system 20 includes control for the objective system movement mechanism 20D.

The objective system movement mechanism 20D includes a rotation mechanism configured to rotate the first ellipsoidal concave mirror 21 and the second ellipsoidal concave mirror 22 around a predetermined rotation axis, and a slide mechanism configured to slide and move the optical system obtained by excluding the objective optical system 20 from the optical system 10, in conjunction with the rotation by the rotation mechanism. Each movement mechanism includes a pulse motor as an actuator, and performs the rotation of the first ellipsoidal concave mirror 21 and the second ellipsoidal concave mirror 22 using the rotation mechanism and the sliding movement of the optical system described above using the slide mechanism, under control from the main controller 101.

Examples of the control for the illumination optical system 30 include control for the light source unit 31, control for the movement mechanism 34D, and control for the optical scanner 95.

Examples of the control for the light source unit 31 include turning on and off, adjustment of the light amount, for each of the visible light sources 312R, 312G, 132B and the infrared light source 312IR.

The movement mechanism 34D moves the slit 34 in the optical axis direction of the illumination optical system 30. The main controller 101 outputs control signal(s) to the movement mechanism 34D to move the slit 34 in a direction corresponding to the control signal(s) by the amount of movement corresponding to the control signal(s).

In addition, the movement mechanism 34D includes a rotation mechanism configured to change the intersection angle of the exit plane of the slit 34 with respect to the optical axis of the illumination optical system 30. The rotation mechanism rotates the slit 34 around a rotation axis perpendicular to the optical axis of the illumination optical system 30. The movement mechanism 34D receives control from the main controller 101 in the left eye photographing mode, and rotates the slit 34 so that the longitudinal direction of the slit 34 is optically parallel to the major axis direction of the first ellipsoidal concave mirror 21 in the first rotational state shown in FIG. 6A. In addition, the movement mechanism 34D receives control from the main controller 101 in the right eye photographing mode, and rotates the slit 34 so that the longitudinal direction of the slit 34 is optically parallel to the major axis direction of the first ellipsoidal concave mirror 21 in the second rotational state shown in FIG. 6B.

In some embodiments, a first slit with an intersection angle described above fixed for the left eye photographing mode and a second slit with an intersection angle described above fixed for the right eye photographing mode are provided. In this case, the movement mechanism 34D is configured to dispose either the first slit or the second slit on the optical axis of the illumination optical system 30, under the control from the main controller 101.

For example, the ophthalmic apparatus 1 is provided with an actuator that generates a driving force for driving the movement mechanism 34D and a transmission mechanism that transmits the driving force from the actuator to the movement mechanism 34D. The actuator includes a pulse motor, for example. The transmission mechanism includes a combination of gears, a rack and pinion, and the like, for example. The movement mechanism 34D receives the driving force generated by the actuator controlled by the main controller 101 from the transmission mechanism, and moves the slit 34 in the optical axis direction or rotates the slit 34 around the rotation axis described above.

The control for the optical scanner 95 includes control of the angle of the deflection surface deflecting the illumination light. By controlling the angle of the deflection surface, the deflection direction (scan direction) of illumination light can be controlled. By controlling an angular range of the deflection surface, the scan range (scan start position and scan end position) can be controlled. By controlling the speed of changing the angle of deflection surface, the scan speed can be controlled.

Examples of control for the optical path switching member 70 includes control of switching optical path in accordance with the photographing mode. The optical path switching member 70 guides the illumination light deflected by the optical scanner 95 to the optical path separating member 50L, under the control from the main controller 101 in the left eye photographing mode. In addition, the optical path switching member 70 guides the illumination light deflected by the optical scanner 95 to the optical path separating member 50R, under the control from the main controller 101 in the right eye photographing mode.

Examples of the control for the photographic optical system 47L include control for the image sensor 40L, and control for the focusing mechanism 40Ld. In addition, examples of the control for the photographic optical system 40R include control for the image sensor 47R, and control for the focusing mechanism 40Rd.

Examples of control for the image sensors 47L and 47R include control of setting the light receivable region on the light receiving surface, and control for reading out the light receiving result(s) using the rolling shutter system (for example, setting of light receiving size corresponding to the size of the illumination pattern, or the like). In addition, examples of the control for the image sensors 47L and 47R include the reset control, the exposure control, the charge transfer control, and the output control.

The focusing mechanisms 40Ld and 40Rd move the photographic optical systems 40L and 40R in the optical axis direction, respectively. The main controller 101 outputs control signal(s) to the focusing mechanisms 40Ld and 40Rd to move the photographic optical systems 40L and 40R in the direction corresponding to the control signal(s) by the amount of movement corresponding to the control signal(s). For example, the ophthalmic apparatus 1 is provided with actuators that generates driving force for driving the focusing mechanisms 40Ld and 40Rd and transmission mechanisms that transmit the driving force from the actuators to the focusing mechanisms 40Ld and 40Rd. The actuator includes a pulse motor, for example. The transmission mechanism includes a combination of gears, a rack and pinion, and the like, for example. The focusing mechanisms 40Ld and 40Rd receive the driving force generated by the actuators controlled by the main controller 101 from the transmission mechanisms, and move the photographic optical systems 40L and 40R in the optical axis direction, respectively.

Examples of control for the fixation projection systems 60L and 60R include control for the fixation light sources 61L and 61R.

Examples of the control for the fixation light sources 61L and 61R include turning on and off of the light source, and adjustment of light amount.

Examples of the control for the anterior segment photographing systems 80L and 80R include control of disposing the light receiving surfaces of the anterior segment cameras 81LL and 81LR at the fundus conjugate positions of the left eye EL, and control of photography of the anterior segment cameras 81LL and 81LR. In addition, examples of the control for the anterior segment photographing systems 80L and 80R include control of disposing the light receiving surfaces of the anterior segment cameras 81RL and 81RR at the fundus conjugate positions of the right eye ER, and control of photography of the anterior segment cameras 81RL and 81RR.

Examples of the control of disposing the light receiving surfaces of the anterior segment cameras 81LL and 81LR at the fundus conjugate positions of the left eye EL include control of moving the anterior segment cameras 81LL and 81LR, or control of moving a focusing lens, that transmits light from the left eye EL, in the optical axis direction. Examples of the control of photography of the anterior segment cameras 81LL and 81LR include control of the light-receptive sensitivity of each camera, control of the frame rate (light receiving timing), and control of synchronizing two cameras.

Examples of the control of disposing the light receiving surfaces of the anterior segment cameras 81RL and 81RR at the fundus conjugate positions of the right eye ER include control of moving the anterior segment cameras 81RL and 81RR, or control of moving a focusing lens, that transmits light from the right eye ER, in the optical axis direction. Examples of the control of photography of the anterior segment cameras 81RL and 81RR include control of the light-receptive sensitivity of each camera, control of the frame rate (light receiving timing), and control of synchronizing two cameras.

The movement mechanism 10D three-dimensionally moves the optical system 10 in FIG. 1 of the ophthalmic apparatus 1 (optical system of apparatus). In a typical example, the movement mechanism 10D includes a mechanism for moving the optical system 10 (body for housing the optical system 10) in the X direction (left-right direction), a mechanism for moving it in the Y direction (up-down direction), and a mechanism for moving it in the Z direction (depth direction, front-back direction, working distance direction). The mechanism for moving in the X direction includes an X stage movable in the X direction and an X movement mechanism for moving the X stage, for example. The mechanism for moving in the Y direction includes a Y stage movable in the Y direction and a Y movement mechanism for moving the Y stage, for example. The mechanism for moving in the Z direction includes a Z stage movable in the Z direction and a Z movement mechanism for moving the Z stage, for example. Each movement mechanism includes a pulse motor as an actuator and operates under the control from the main controller 101.

The control for the movement mechanism 10D is used for alignment and tracking. Here, tracking is to move the optical system of apparatus according to the eye movement of the eye to be examined to be photographed. To perform tracking, alignment and focus adjustment are performed in advance. The tracking is a function of maintaining a suitable positional relationship in which alignment and focusing are matched by causing the position of the optical system of the apparatus and the like to follow the eye movement.

In the case of manual alignment, a user operates the UI unit 220 to relatively move the optical system 10 and the eye to be examined so as to cancel the displacement of the eye to be examined, which is to be photographed, with respect to the optical system. For example, the main controller 101 controls the movement mechanism 10D to relatively move the optical system relative to the eye to be examined, by outputting a control signal corresponding to the operation content for the UI unit 220 to the movement mechanism 10D.

In the case of automatic alignment, the main controller 101 controls the movement mechanism 10D to relatively move the optical system relative to the eye to be examined so as to cancel the displacement of the eye to be examined, which is to be photographed, relative to the optical system. Specifically, as described in Japanese Unexamined Patent Application Publication No. 2013-248376, arithmetic processing using a trigonometric method based on the positional relationship between the two anterior cameras in the anterior segment photographing system 80L or the anterior segment photographing system 80R and the eye to be examined is performed. The main controller 101 controls the movement mechanism 10D so that the positional relationship of the eye to be examined relative to the optical system becomes a predetermined relationship.

Examples of the control for the image forming unit 200 include image forming control of forming images of the left eye EL or the right eye ER from the light receiving result(s) obtained using the image sensor 47L or the image sensor 47R.

Examples of control for the data processor 210 include control of image processing for the images acquired by the photographic optical systems 40L and 40R, control of analysis processing for the images acquired by the anterior segment photographing systems 80L and 80R, and a control of position matching (alignment control) of the optical system of apparatus relative to the eye to be examined.

Examples of the control for the UI unit 220 include control for a display device and control for an operation device (input device).

### (Storage unit 102)

The storage unit 102 stores various types of data. Examples of the data stored in the storage unit 102 include light receiving result(s) obtained by the image sensors 47L and 47R, image data of an image formed by the image forming unit 200, processing results obtained by the data processor 210, and information on eye to be examined. The information on the eye to be examined includes information on the examinee such as patient ID and name, and information on the eye to be examined such as identification information of the left/right eye.

In addition, the storage unit 102 stores various kinds of computer programs and data for operating the ophthalmic apparatus 1.

### (Image forming unit 200)

The image forming unit 200 can form the light receiving image corresponding to the arbitrary opening range based on the light receiving result(s) read out from the image sensor 47L or the image sensor 47R using the rolling shutter system under the control from the main controller 101 (controller 100). The image forming unit 200 can sequentially form light receiving light images corresponding to the (virtual) opening ranges and can form an image of the left eye EL or the right eye ER from a plurality of formed light receiving images. The various images (the various image data) formed by the image forming unit 200 are stored in the storage unit 102, for example.

For example, the image forming unit 200 includes a processor, and executes the function described above by performing processing corresponding to the program(s) stored in the storage unit or the like.

### (Data processor 210)

The data processor 210 performs various kinds of image processing, various kinds of analysis processing on the light receiving result(s) obtained by the image sensors 47L and 47R, and alignment processing. Examples of the image processing include noise removal processing on the light receiving results, brightness correction processing for easily identifying a predetermined site depicted in the light receiving image based on the light receiving results. Examples of the alignment processing include processing for perform position matching of the optical system of apparatus with respect to the eye to be examined.

The data processor 210 includes a processor, and realizes the above functions by performing processing corresponding to the program(s) stored in the storage unit or the like.

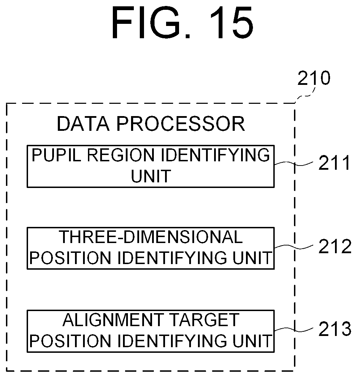

FIG. 15 shows a block diagram of an example of the configuration of the data processor 210 of FIG. 14.

The data processor 210 includes a pupil region identifying unit 211, a three-dimensional position identifying unit 212, and an alignment target position identifying unit 213.

The pupil region identifying unit 211 acquired a pair of anterior segment images (photographic images) of the left eye EL obtained by photographing the left eye EL substantially simultaneously using the anterior segment cameras 81LL and 81LR of the anterior segment photographing system 80L. The pupil region identifying unit 211 identifies a position (center position, position of the center of gravity) of a pupil region in the anterior segment image corresponding to the pupil of the left eye EL, by analyzing each of the acquired pair of the anterior segment images.

First, the pupil region identifying unit 211 identifies an image region (pupil region) corresponding to the pupil of the left eye EL, based on the distribution of pixel values (luminance values etc.) in the anterior segment image. Generally, the pupil is represented with lower luminance compared to other sites, and therefore, the pupil region may be identified by searching an image region with low luminance. In this case, the pupil region may be identified by taking the shape of the pupil into consideration. That is, it is possible to configure such that the pupil region is identified by searching for a substantially circular image region with low luminance.

Next, the pupil region identifying unit 211 identifies the center position of the identified pupil region. As described above, the pupil is substantially circular; therefore, it is possible to identify the contour of the pupil region, to identify the center position of this contour (an approximate circle or an approximate ellipse thereof), and to treat this as the pupil center position. Instead, by obtaining the center of gravity of the pupil region, this position may be used as the position of the center of gravity of the pupil.

Similarly, the pupil region identifying unit 211 acquired a pair of anterior segment images (photographic images) of the right eye ER obtained by photographing the right eye ER substantially simultaneously using the anterior segment cameras 81RL and 81RR of the anterior segment photographing system 80R. The pupil region identifying unit 211 identifies a position of a pupil region in the anterior segment image corresponding to the pupil of the right eye ER, in the same way as the pupil region of the left eye EL, by analyzing each of the acquired pair of the anterior segment images.

The pupil region identifying unit 211 can sequentially identify the pupil regions corresponding to the pupil for the pair of the anterior segment images sequentially obtained by the anterior segment cameras 81LL and 81LR or the anterior segment cameras 81RL and 81RR. In addition, the pupil region identifying unit 211 may identify the pupil regions every one or more arbitrary number of frames for the pair of the anterior segment images sequentially obtained by the anterior segment cameras 81LL and 81LR or the anterior segment cameras 81RL and 81RR.

The three-dimensional position identifying unit 212 identifies the tree-dimensional position of the pupil of the left eye EL based on the positions of the anterior segment cameras 81LL and 81LR and the pupil region (center position) identified by the pupil region identifying unit 211. The three-dimensional position identifying unit 212 applies a known trigonometry to the positions of the two anterior segment cameras 81LL and 81LR (these are known) and the positions corresponding to the pupil regions in the pair of the anterior segment images, as disclosed in Japanese Unexamined Patent Application Publication No. 2013-248376. Thereby, the three-dimensional position identifying unit 212 can calculate the thee-dimensional position of the pupil of the left eye EL as the three-dimensional position of the left eye EL.

In addition, the three-dimensional position identifying unit 212 identifies the tree-dimensional position of the pupil of the right eye ER based on the positions of the anterior segment cameras 81RL and 81RR and the pupil region (center position) identified by the pupil region identifying unit 211. The three-dimensional position identifying unit 212 calculates the three-dimensional position of the pupil of the right eye ER as the three-dimensional position of the right eye ER, by applying a known trigonometry to the positions of the two anterior segment cameras 81RL and 81RR (these are known) and the positions corresponding to the pupil regions in the pair of the anterior segment images.

The alignment target position identifying unit 213 identifies an alignment target position (Xr, Yr, Zr). The alignment target position (Xr, Yr, Zr) is a three-dimensional position defined in a three-dimensional coordinate system with the origin at a predetermined reference position in the optical system of the ophthalmic apparatus 1. The coordinate position Xr in the X direction and the coordinate position Yr in the Y direction of the alignment target position are the positions on the XY plane where the optical axis of the photographic optical system 40L approximately coincides with the axis of the left eye EL or the positions on the XY plane where the optical axis of the photographic optical system 40R approximately coincides with the axis of the right eye ER. The coordinate position Zr in the Z direction of the alignment target position is the position on the optical axis of the photographic optical system 40L where the distance from the photographic optical system 40L to the left eye EL becomes a predetermined working distance or the position on the optical axis of the photographic optical system 40R where the distance from the photographic optical system 40R to the right eye ER becomes a predetermined working distance. Here, the working distance is a preset value which is called a working distance of the objective optical system 20, and it means the distance between the eye to be examined and the optical system 10 when measuring (photographing) using the photographic optical system 40L or 40R.

The main controller 101 controls the movement mechanism 10D so as to dispose the eye to be examined, which is to be photographed, at the alignment target position calculated by the alignment target position identifying unit 213.

### (UI unit 220)

The UI unit 220 has a function for exchanging information between a user (examiner or examinee) and the ophthalmic apparatus 1. The UI unit 220 includes the display device and the operation device. The display device may include a display unit, and it may include another display device. The display device displays various information. The display device includes a liquid crystal display, for example. The display device displays the above information under the control of the main controller 101. Examples of the information displayed on the display device include information corresponding to the control result by the controller 100, information (image) corresponding to the calculation result by the image forming unit 200 or the data processor 210. The operation device includes various hardware keys and/or various software keys. Upon receiving the operation content for the operation device, the main controller 101 can output a control signal corresponding to the operation content to each part of the fundus imaging apparatus. At least a part of the display device and at least a part of the operation device may be configured integrally. One example of this is the touch panel display.

<Operation example>

Next, the operation of the ophthalmic apparatus 1 according to the embodiments will be described.

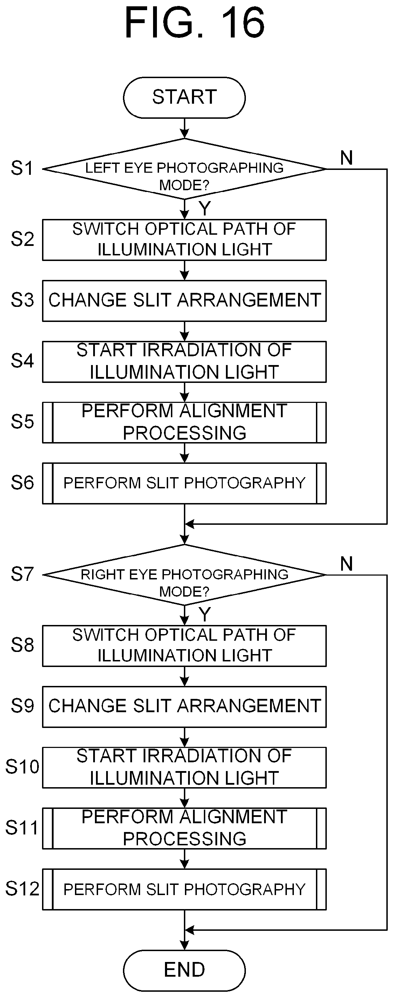

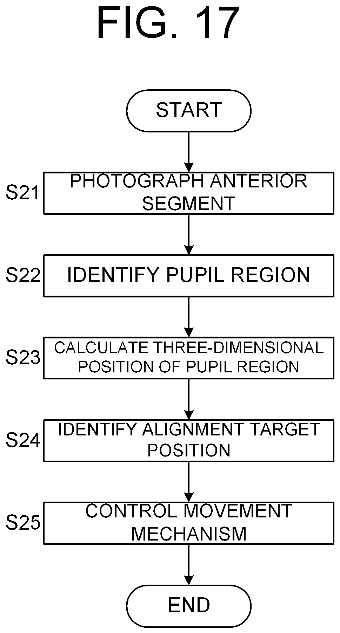

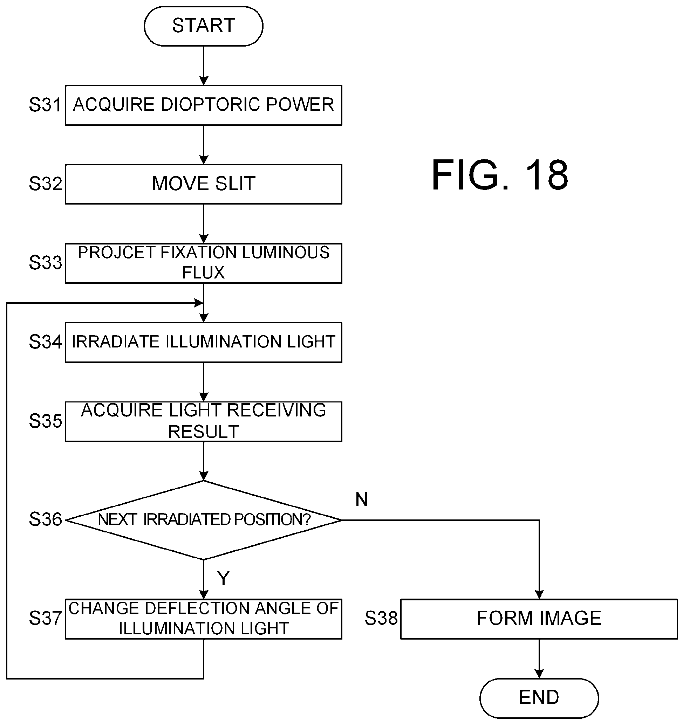

FIG. 16 , FIG. 17, and FIG. 18 show examples of an operation of the ophthalmic apparatus 1 according to the embodiments. FIG. 16 represents a flowchart of an example of the operation of the ophthalmic apparatus 1 according to the embodiments. FIG. 17 represents a flowchart of an example of the operation of step S5 and step S11 in FIG. 16. FIG. 18 represents a flowchart of an example of the operation of step S6 and step S12 in FIG. 16. The storage unit 102 stores computer programs for realizing the processing shown in FIGs. 16 to 18. The main controller 101 operates according to the computer programs, and thereby the main controller 101 performs the processing shown in FIGs. 16 to 18.

### (S1: Left eye photographing mode?)

First, the main controller 101 determines whether or not the photographing mode is the left eye photographing mode. For example, the main controller 101 determines the type of the photographing mode based on the operation content input by the user through the UI unit 220.

When it is determined that the photographing mode is the left eye photographing mode (S1: Y), the operation of the ophthalmic apparatus 1 proceeds to step S2, and slit photography control is performed on the left eye EL in steps S2 to S6. When it is determined that the photographing mode is not the left eye photographing mode (S1: N), the operation of the ophthalmic apparatus 1 proceeds to step S7.

### (S2: Switch optical path of illumination light)

When it is determined in step S1 that the photographing mode is the left eye photographing mode (S1: Y), the main controller 101 controls the optical path switching member 70 to switch the optical path so that the illumination light is guided to the optical path separating member 50L.

### (S3: Change slit arrangement)

Subsequently, the main controller 101 controls the movement mechanism 34D to change the intersection angle of the slit 34 relative to the optical axis so that the exit plane of the slit 34 is optically approximately parallel to the major axis direction and the first ellipsoidal concave mirror 21 shown in FIG. 6A.

### (S4: Starting irradiation of illumination light)

Subsequently, the main controller 101 controls the light source unit 31 to start irradiation of the illumination light. For example, the light source unit 31 starts irradiating white light or infrared light as the illumination light.

### (S5: Perform alignment processing)

Next, the main controller 101 perform alignment processing of the optical system 10 with respect to the left eye EL using the anterior segment photographing system 80L. The details of step S5 will be described below.

### (S6: Perform slit photography)

Subsequently, the main controller 101 performs photography processing on the fundus of the left eye EL with a slit scan system. The details of step S6 will be described below.

### (S7: Right eye photographing mode?)

When it is determined in the step S1 that the photographing mode is not the left eye photographing mode (S1: N), or subsequent to step S6, the main controller 101 determines whether or not the photographing mode is the right eye photographing mode. For example, the main controller 101 determines the type of the photographing mode based on the operation content input by the user through the UI unit 220.

When it is determined that the photographing mode is the right eye photographing mode (S7: Y), the operation of the ophthalmic apparatus 1 proceeds to step S8, and slit photography control is performed on the right eye ER in steps S8 to S12. When it is determined that the photographing mode is not the right eye photographing mode (S7: N), the ophthalmic apparatus 1 terminates the operation (END).

### (S8: Switch optical path of illumination light)

When it is determined in step S7 that the photographing mode is the right eye photographing mode (S7: Y), the main controller 101 controls the optical path switching member 70 to switch the optical path so that the illumination light is guided to the optical path separating member 50R.

### (S9: Change slit arrangement)

Subsequently, the main controller 101 controls the movement mechanism 34D to change the intersection angle of the slit 34 relative to the optical axis so that the exit plane of the slit 34 is optically approximately parallel to the major axis direction and the first ellipsoidal concave mirror 21 shown in FIG. 6B.

### (S10: Starting irradiation of illumination light)

Subsequently, the main controller 101 controls the light source unit 31 to start irradiation of the illumination light, in the same way as in step S4.

### (S11: Perform alignment processing)

Next, the main controller 101 perform alignment processing of the optical system 10 with respect to the right eye ER using the anterior segment photographing system 80R. Step S11 is similar to step S5, except that anterior segment photographing system 80R is used instead of the anterior segment photographing system 80L.

### (S12: Perform slit photography)

Subsequently, the main controller 101 performs photography processing on the fundus of the right eye ER with a slit scan system. Step S12 is similar to step S6, except that photographic optical system 40R is used instead of the photographic optical system 40L.

This terminates the operation of the ophthalmic apparatus 1 (END).

The processing in step S5 or step S11 of FIG. 16 is performed according to the flow shown in FIG. 17. In FIG. 17, the processing of step S5 will be described for the left eye EL. However, a detailed description of the processing of step S11 is omitted for the right eye ER, since the processing of step S11 is also similar.

In FIG. 17, it is assumed that the anterior segment cameras 81LL and 81LR of the anterior segment photographing system 80L are already disposed at the fundus conjugate position of the left eye EL, prior to step S21.

### (S21: Photograph anterior segment)

In step S5, first, the main controller 101 controls the anterior segment cameras 81LL and 81LR in the anterior segment photographing system 80L to start photographing the anterior segment of the left eye EL from different directions, and starts acquiring a pair of anterior segment images acquired substantially simultaneously.

### (S22: Identify pupil region)

Next, the main controller 101 controls the pupil region identifying unit 211 to identify the pupil region for each of the pair of anterior segment images acquired in step S21.

### (S23: Calculate three-dimensional position of pupil region)

Next, the main controller 101 controls the three-dimensional position identifying unit 212 to calculate the three-dimensional position of the pupil region of the left eye EL using the pupil regions in the pair of anterior segment images identified in step S22, as described above.

### (S24: Identify alignment target position)

Subsequently, the main controller 101 controls the alignment target position identifying unit 213 to identify the alignment target position based on the three-dimensional position of the pupil region calculated in step S23, as described above.

### (S25: Control movement mechanism)

Next, the main controller 101 controls the movement mechanism 10D to relatively move the optical system 10 relative to the left eye EL based on the alignment target position calculated in step S25.

This terminates the processing of step S5 (END).

The processing in step S6 or step S12 of FIG. 16 is performed according to the flow shown in FIG. 18. In FIG. 18, the processing of step S6 will be described for the left eye EL. However, a detailed description of the processing of step S12 is omitted for the right eye ER, since the processing of step S12 is also similar.

### (S31: Acquire dioptric power)

In step S6, first, the main controller 101 acquires the dioptric power (refractive power). For example, the main controller 101 acquires the dioptric power of the left eye EL from an external ophthalmic measurement apparatus or an electronic medical record. In some embodiments, the main controller 101 controls the focusing mechanism 40Ld to identify the focusing state, and identifies the dioptric power from a position on the axis of the photographic optical system 40L, which is set to the focusing state (or the control result for the actuator that drives the focusing mechanism 40Ld).

### (S32: Move slit)

Next, the main controller 101 changes the position of the slit 34 on the optical axis of the illumination optical system 30 in accordance with the dioptric power of the left eye EL acquired in step S31.

Specifically, the main controller 101 specifies the position of the slit 34 corresponding to the dioptric power by referring to the first control information stored in the storage unit 102, and controls the movement mechanism 34D so as to dispose the slit 34 at the identified position.

### (S33: Project fixation luminous flux)

Subsequently, the main controller 101 controls the fixation projection system 60L to start projecting the fixation luminous flux onto the fundus of the left eye EL.

### (S34: Irradiate illumination light)

Next, the main controller 101 starts the deflection control of the optical scanner 95 for the slit-shaped illumination light generated by the illumination optical system 30, and starts irradiating the illumination light onto the desired irradiated region on the fundus of the left eye EL. When the irradiation of the illumination light is started, the slit-shaped illumination light is sequentially irradiated within the desired irradiated range as described above.

### (S35: Acquire light receiving result)

The main controller 101 acquires the light receiving result(s) of the pixels in the opening range of the image sensor 47L corresponding to the irradiated range of the illumination light on the fundus in step S34, as described above.

### (S36: Next irradiated position?)

The main controller 101 determines whether or not the irradiated position to be irradiated next with the illumination light is present. The main controller 101 can determine whether or not the irradiated position to be irradiated next with the illumination light is present, by determining whether or not the irradiated ranges of the illumination light that is moved sequentially has covered a predetermined photographing range of the fundus.

When it is determined that the irradiated position to be irradiated next with the illumination light is present (S36: Y), the operation of the ophthalmic apparatus 1 proceeds to step S37. When it is determined that the irradiated position to be irradiated next with the illumination light is not present (S36: N), the operation of the ophthalmic apparatus 1 proceeds to step S38.

### (S37: Change deflection angle of illumination light)

When it is determined in step S36 that the irradiated position to be irradiated next with the illumination light is present (S36: Y), the main controller 101 controls the optical scanner 95 to change the deflection angle of the deflection surface of the optical scanner 95 by a predetermined angle.

Subsequent to step S37, the processing of step S6 proceeds to step S34.

### (S38: Form image)

When it is determined in step S36 that the irradiated position to be irradiated next with the illumination light is not present (S36: N), the main controller 101 controls the image forming unit 200 to form the fundus image of the left eye EL from the light receiving results acquired repeatedly while changing the irradiated range of the illumination light in steps S34 to S37.

For example, the image forming unit 200 syntheses a plurality of light receiving results with different irradiated ranges (opening range on the light receiving surface of the image sensor 47L) of the illumination light for the number of times repeating the process in steps S34 to S37, based on the order of the movement of the irradiated range. Thereby, the fundus image of the left eye EL for one frame is formed.

In some embodiments, in step S34, the illumination light is irradiated on the irradiated range set so as to have an overlapping region with the adjacent irradiated range. Thereby, in step S38, the fundus image for one frame is formed by synthesizing the images of irradiated ranges so as to overlap the overlapping region with each other.

This terminates the processing of step S6 in FIG. 16 (END).

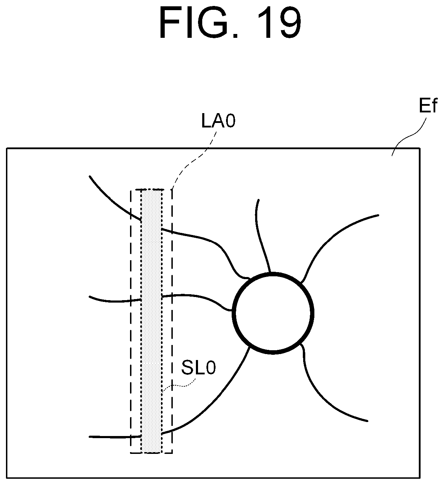

FIG. 19 schematically shows the fundus of the eye to be examined, which is to be photographed.

The slit image (image of the aperture formed in slit 34) SL0 is projected onto the fundus Ef of the left eye EL or the right eye ER, which is to be photographed. In the image sensor 47L or the image sensor 47R, the position of the virtual light receivable region LA0 on the light receiving surface is changed in synchronization with the movement of the slit image corresponding to the illuminated region of the illumination light on the fundus Ef.

Specifically, the light receivable region LA0 of the image sensor 47L or the image sensor 47R is set so as to include a range of the slit image SL0 on the fundus Ef. The fundus Ef is scanned so that the slit image SL0 moved in the shorter direction perpendicular to the longitudinal direction of the slit image SL0. The image sensor 47L or the image sensor 47R sequentially moves the light receivable region LA0 on the light receiving surface so as to include a range of the slit image SL0, in synchronization with the movement of the slit image SL0 on the fundus Ef.

In the embodiments described above, a case where the objective optical system 20 is shared with the left eye photographing mode and the right eye photographing mode. However, the configuration according to the embodiments is not limited thereto. For example, an objective optical system for the left eye photographing mode and an objective optical system for the right eye photographing mode may be provided. In this case, two ellipsoidal concave mirrors (for example, the first ellipsoidal concave mirror 21 and the second ellipsoidal concave mirror 22) are provided for the photography or the measurement of the left eye EL, and two ellipsoidal concave mirrors (for example, the first ellipsoidal concave mirror 21' and the second ellipsoidal concave mirror 22') are provided for the photography or the measurement of the right eye ER.

In the embodiments described above, the photographic optical systems 40L and 40R may be a single photographic optical system common to by the left eye EL and the right eye ER. Further, the fixation projection systems 60L and 60R may be a single fixation projection system common to the left eye EL and the right eye ER. The anterior segment photographing systems 80L and 80R may be a single anterior segment photographing system common to the left eye EL and the right eye ER.

In addition, in the embodiments described above, the two or more cameras in each anterior segment photographing system do not need to have the same angle formed between the measurement conjugate plane and the photographic optical axis at a position substantially conjugate optically to the measurement position. Further, the two or more cameras may be arranged so as to be asymmetric with reference to the normal direction of the measurement conjugate surface (so that the angles formed between the normal direction and the photographic axis are different).

Furthermore, in the embodiments described above, a case where the dichroic mirror 90L or the dichroic mirror 90R is disposed between the first ellipsoidal concave mirror 21, and the optical path separating member 50L or the optical path separating member 50R has been described. However, the embodiments is not limited to this. For example, the dichroic mirror 90L may be disposed between the first ellipsoidal concave mirror 21 and the second ellipsoidal concave mirror 22 shown in FIG. 6A in the left eye photographing mode. In this case, the anterior segment photographing system 80L can be disposed so as to view the second focal point F2 (primary left eye measurement conjugate position) of the first ellipsoidal concave mirror 21. Similarly, for example, the dichroic mirror 90R may be disposed between the first ellipsoidal concave mirror 21 and the second ellipsoidal concave mirror 22 shown in FIG. 6B in the right eye photographing mode. In this case, the anterior segment photographing system 80R can be disposed so as to view the second focal point F2' (primary right eye measurement conjugate position) of the first ellipsoidal concave mirror 21.

### [Actions]

The ophthalmic apparatus according to the embodiments will be described.

The first aspect of embodiments is an ophthalmic apparatus (1) including an objective optical system (20), an illumination optical system (30), a photographic optical system (40L, 40R), a photographing unit (anterior segment photographing system 80L, 80R), a movement mechanism (10D), and a three-dimensional position identifying unit (212). The objective optical system is configured to optically relay a measurement position (left eye measurement position, right eye measurement position) where a pupil of an eye (left eye EL, right eye ER) of an examinee can be disposed. The illumination optical system is configured to irradiate illumination light onto the eye through the objective optical system. The photographic optical system is configured to receive returning light of the illumination light from the eye through the objective optical system. The photographing unit includes two or more cameras (anterior segment cameras 81LL and 81LR, or anterior segment cameras 81RL and 81RR) disposed so as to include a position (left eye measurement conjugate position, right eye measurement conjugate position), that is substantially conjugate optically to the measurement position relayed by the objective optical system, within a field of vision. The movement mechanism is configured to relatively move the objective optical system, the illumination optical system, the photographic optical system, and the photographing unit relative to the eye. The three-dimensional position identifying unit is configured to identify a three-dimensional position of the eye based on two or more photographic images of the eye acquired by the photographing unit.

According to such an aspect, the measurement position, where the pupil of the eye of the examinee can be disposed, can be optically relayed by the objective optical system, and the photographing unit including the two or more cameras can be disposed so as to view the position substantially conjugate optically to the measurement position. Thereby, even when a distance between the eye and the objective optical system is short, the two or more photographic images of the eye can be acquired, and the position matching between the optical system and the eye to be examined can be preferably performed based on the acquired two or more photographic images. As a result, high-definition photographing or high-precision measurement of the eye to be examined at a wide angle can be performed.

In the second aspect of the embodiments, in the first aspect, the two or more cameras are disposed so that angles formed by a conjugate plane, that is substantially conjugate optically to the measurement position, and photographic optical axes of the cameras are the same, and that the angles are symmetrical with reference to a normal direction of the conjugate plane.

This can reduce the processing load required to identify the three-dimensional position of the eye of the examinee. Thus, even when a distance between the eye and the objective optical system is short, high-definition photographing or high-precision measurement of the eye of the examinee at a wide angle can be performed, while reducing the processing load.

The third aspect of the embodiments, in the first aspect or the second aspect, further includes a first optical path separating member (optical path separating member 50L, 50R), and a second optical path separating member (dichroic mirror 90L, 90R). The first optical path separating member is disposed at a position substantially conjugate optically to the measurement position (left eye measurement conjugate position, right eye measurement conjugate position), and is configured to separate an optical path of the illumination light and an optical path of the returning light. The second optical path separating member is disposed between the objective optical system and the first optical path separating member, and is configured to guide at least part of light from the eye to the photographing unit.

According to such an aspect, at least part of light from the eye is configured to be guided to the photographing unit between the objective optical system and the first optical path separating member. Thereby, the configuration accompanying the addition of the optical system for position matching between the eye of the examinee and the optical system can be simplified.

**In** the fourth aspect of the embodiments, in any one of the first aspect to the third aspect, the first optical path separating member includes a reflecting member (51L, 51R), and a photographic diaphragm (52L, 52R). The reflecting member is disposed at a position substantially conjugate optically to the measurement position (left eye measurement conjugate position, right eye measurement conjugate position). The photographic diaphragm is disposed at a position substantially conjugate optically to the measurement position, and has an aperture formed at a position eccentric to an optical axis. The reflecting member is configured to reflect illumination light from the illumination optical system to guide the illumination light to the objective optical system. The photographic diaphragm is configured to guide the returning light passing through the aperture formed in the photographic diaphragm to the photographic optical system.

According to such an aspect, the optical path of the illumination optical system and the optical path of the photographic optical system are separated using the reflecting member and the photographic diaphragm. Thereby, the size of the optical system can be reduced with a simple configuration.

**In** the fifth aspect of the embodiments, in the third aspect, the second optical path separating member includes a beam splitter (dichroic mirror 90L, 90R).

According to such an aspect, at least part of the light from the eye can be guided to the photographing unit with a simple configuration.

**In** the sixth aspect of the embodiments, in the fourth aspect, the illumination optical system includes an illumination diaphragm (iris aperture 32). The illumination diaphragm is disposed at a position substantially conjugate optically to the measurement position (left eye measurement conjugate position, right eye measurement conjugate position), and has an aperture formed at a position eccentric to an optical axis. An image of the aperture formed in the photographic diaphragm and an image of the aperture formed in the illumination diaphragm are non-overlapping at a position substantially conjugate optically to the measurement position.

According to such an aspect, the illumination light, which enters the eye of the examinee, and the returning light from the illumination light, which emits from the eye, can be divided on the pupil. Thereby, wide-angle images of the eye of the examinee can be acquired while reducing the size of the optical system.

The seventh aspect of the embodiments, in any one of the first aspect to the sixth aspect, further includes a controller (100, main controller 101) configured to control the movement mechanism based on the three-dimensional position.

According to such an aspect, automatic alignment can be performed. Thereby, high-definition photographing or high-precision measurement of the eye of the examinee at a wide angle can be performed.

**In** the eighth aspect of the embodiments, in any one of the first aspect to the seventh aspect, the objective optical system includes a concave mirror (first ellipsoidal concave mirror 21, second ellipsoidal concave mirror 22) having a concave-shaped reflective surface.

According to such an aspect, the ophthalmic apparatus that can perform high-definition photographing or high-precision measurement of the eye of the examinee at a wide angle using the concave mirror can be provided.

In the ninth aspect of the embodiments, in any one of the first aspect to the eighth aspect, the objective optical system is configured to optically relay a left eye measurement position where a pupil of a left eye (EL) of the examinee can be disposed, and to optically relay a right eye measurement position where a pupil of a right eye (ER) of the examinee can be disposed. The photographing unit includes a photographing unit for left eye (anterior segment photographing system 80L) and a photographing unit for right eye (anterior segment photographing system 80R). The photographing unit for left eye includes two or more cameras (anterior segment cameras 81LL and 81LR) disposed so as to include a position (left eye measurement conjugate position), that is substantially conjugate optically to the left eye measurement position relayed by the objective optical system, within a field of vision. The photographing unit for right eye includes two or more cameras (anterior segment cameras 81RL and 81RR) disposed so as to include a position (right eye measurement conjugate position), that is substantially conjugate optically to the right eye measurement position relayed by the objective optical system, within a field of vision.

According to such an aspect, the photographing unit for left eye and the photographing unit for right eye are provided for performing position matching on the left eye and right eye of the examinee, respectively. Thereby, high-precision position matching can be performed on each of the left eye and the right eye. As a result, the ophthalmic apparatus, that can perform high-definition photographing or high-precision measurement at a wide angle on each of the left eye and right eye of the examinee, can be provided.

In the tenth aspect of the embodiments, in the ninth aspect, the illumination optical system is configured to irradiate illumination light onto the left eye through the objective optical system, and to irradiate illumination light onto the right eye through the objective optical system. The photographic optical system includes a photographic optical system for left eye (photographic optical system 40L) and a photographic optical system for right eye (photographic optical system 40R). The photographic optical system for left eye is configured to receive returning light of the illumination light from the left eye through the objective optical system. The photographic optical system for right eye is configured to receive returning light of the illumination light from the right eye through the objective optical system.

According to such an aspect, the objective optical system is shared with the left eye and the right eye, and the photographic optical system is provided for each of the left eye and the right eye. Thereby, high-definition photographing or high-precision measurement at a wide angle can be performed on each of the left eye and right eye of the examinee, while reducing the size of the optical system.

In the eleventh aspect of the embodiments, in the tenth aspect, the illumination optical system is configured to sequentially irradiate illumination light onto the left eye and the right eye through the objective optical system.

According to such an aspect, the illumination optical system can be shared with the left eye and the right eye. Thereby, the size of the optical system can be reduced.

In the twelfth aspect of the embodiments, in the ninth aspect, the objective optical system includes two concave mirrors (first ellipsoidal concave mirror 21 and second ellipsoidal concave mirror 22) configured to be capable of rotating around a predetermined rotation axis (Ra). The two concave mirrors are configured to optically relay the left eye measurement position in a first rotational state around the predetermined rotation axis, and to optically relay the right eye measurement position in a second rotational state around the predetermined rotation axis.

According to such an aspect, high-definition photographing or high-precision measurement at a wide angle can be performed on each of the left eye and right eye of the examinee, while suppressing the increase in size of the optical system.

In the thirteenth aspect of the embodiments, in the twelfth aspect, the two concave mirrors include a first ellipsoidal concave mirror (21) and a second ellipsoidal concave mirror (22). A second focal point (F2, F2') of the first ellipsoidal concave mirror is disposed at a first focal point (F3, F3') of the second ellipsoidal concave mirror. The left eye measurement position or the right eye measurement position can be disposed at a second focal point (F4, F4') of the second ellipsoidal concave mirror. The ophthalmic apparatus further includes an optical path separating member for left eye (optical path separating member 50L), and an optical path separating member for right eye (optical path separating member 50R). The optical path separating member for left eye is disposed at a first focal point (F1, F1') of the first ellipsoidal concave mirror in the first rotational state, and is configured to separate an optical path of illumination light irradiated onto the left eye and an optical path of returning light of the illumination light from the left eye. The optical path separating member for right eye is disposed at the first focal point of the first ellipsoidal concave mirror in the second rotational state, and is configured to separate an optical path of illumination light irradiated onto the right eye and an optical path of returning light of the illumination light from the right eye.

According to such an aspect, the ophthalmic apparatus that can perform high-definition photographing or high-precision measurement at a wide angle using the two concave mirrors can be provided.

In the fourteenth aspect of the embodiments, in the thirteenth aspect, the illumination optical system includes an optical path switching member (70) configured to guide illumination light to the optical path separating member for left eye or the optical path separating member for right eye.

According to such an aspect, the illumination optical system can be shared with the left eye and the right eye with a simple configuration. Thereby, the size of the optical system can be reduced.

The fifteenth aspect of the embodiments, in any one of the twelfth aspect to the fourteenth aspect, further includes a slide mechanism (objective system movement mechanism 20D). The slide mechanism is configured to move the objective optical system, the illumination optical system, the photographic optical system, and the photographing unit in a direction intersecting the predetermined rotation axis, in conjunction with a rotation of the two concave mirrors.

According to such an aspect, when switching between photographing (measuring) the left eye and photographing (measuring) the right eye, the amount of change in the direction of the face of the examinee can be minimized. Thereby, high-definition photographing or high-precision measurement at a wide angle can be performed on each of the left eye and right eye of the examinee, while reducing the burden on the examinee.

In the sixteenth aspect of the embodiments, in any one of the first aspect to the fifteenth aspect, the illumination optical system is configured to generate slit-shaped illumination light, and to deflect the slit-shaped illumination light. The photographic optical system is configured to receive the returning light in a light receiving range corresponding to an irradiated range of the slit-shaped illumination light on the eye.

According to such an aspect, the returning light of the illumination light can be received without being affected by unnecessary light. Thereby, higher-definition photographing or higher-precision measurement at a wide angle can be performed on the eye of the examinee.

In the seventeenth aspect of the embodiments, in the sixteenth aspect, the photographic optical system is configured to capture light receiving result of the returning light using a rolling shutter system.

According to such an aspect, higher-definition photographing or higher-precision measurement at a wide angle can be performed on the eye of the examinee with a simple configuration.

### < Others >

The embodiment described above is merely an example for implementing the present invention. Those who intend to implement the present invention can apply any modification, omission, addition, or the like within the scope of the gist of the present invention.

### EXPLANATION OF SYMBOLS

1 Ophthalmic apparatus
10 Optical system
10D Movement mechanism
20 Objective optical system
20D Objective system movement mechanism
21 First ellipsoidal concave mirror
22 Second ellipsoidal concave mirror
30 Illumination optical system
31 Light source unit
32 Iris aperture
34 Slit
40L, 40R Photographic optical system
47L, 47R Image sensor
50L, 50R Optical path separating member
60L, 60R Fixation projection system
70 Optical path switching member
80L, 80R Anterior segment photographing system
81LL, 81LR, 81RL, 81RR Anterior segment camera
90L, 90R Dichroic mirror
95 Optical scanner
100 Controller
101 Main controller
102 Storage unit
200 Image forming unit
210 Data processor
211 Pupil region identifying unit
212 Three-dimensional position identifying unit
213 Alignment target position identifying unit
BSL, BSR Beam splitter
EL Left eye
ER Right eye
F1, F3 First focal point
F2, F4 Second focal point

## Claims

1. An ophthalmic apparatus, comprising:
an objective optical system configured to optically relay a measurement position where a pupil of an eye of an examinee can be disposed;
an illumination optical system configured to irradiate illumination light onto the eye through the objective optical system;
a photographic optical system configured to receive returning light of the illumination light from the eye through the objective optical system;
a photographing unit including two or more cameras disposed so as to include a position, that is substantially conjugate optically to the measurement position relayed by the objective optical system, within a field of vision;
a movement mechanism configured to relatively move the objective optical system, the illumination optical system, the photographic optical system, and the photographing unit relative to the eye; and
a three-dimensional position identifying unit configured to identify a three-dimensional position of the eye based on two or more photographic images of the eye acquired by the photographing unit.

2. The ophthalmic apparatus of claim 1, wherein
the two or more cameras are disposed so that angles formed by a conjugate plane, that is substantially conjugate optically to the measurement position, and photographic optical axes of the cameras are the same, and that the angles are symmetrical with reference to a normal direction of the conjugate plane.

3. The ophthalmic apparatus of claim 1 or 2, further comprising:
a first optical path separating member disposed at a position substantially conjugate optically to the measurement position, and configured to separate an optical path of the illumination light and an optical path of the returning light; and
a second optical path separating member disposed between the objective optical system and the first optical path separating member, and configured to guide at least part of light from the eye to the photographing unit.

4. The ophthalmic apparatus of claim 3, wherein
the first optical path separating member includes:
a reflecting member disposed at a position substantially conjugate optically to the measurement position; and
a photographic diaphragm disposed at a position substantially conjugate optically to the measurement position, and having an aperture formed at a position eccentric to an optical axis, wherein
the reflecting member is configured to reflect illumination light from the illumination optical system to guide the illumination light to the objective optical system, and
the photographic diaphragm is configured to guide the returning light passing through the aperture formed in the photographic diaphragm to the photographic optical system.

5. The ophthalmic apparatus of claim 3, wherein
the second optical path separating member includes a beam splitter.

6. The ophthalmic apparatus of claim 4, wherein
the illumination optical system includes an illumination diaphragm disposed at a position substantially conjugate optically to the measurement position and having an aperture formed at a position eccentric to an optical axis, wherein
an image of the aperture formed in the photographic diaphragm and an image of the aperture formed in the illumination diaphragm are non-overlapping at a position substantially conjugate optically to the measurement position.

7. The ophthalmic apparatus of claim 1 or 2, further comprising
a controller configured to control the movement mechanism based on the three-dimensional position.

8. The ophthalmic apparatus of claim 1 or 2, wherein
the objective optical system includes a concave mirror having a concave-shaped reflective surface.

9. The ophthalmic apparatus of claim 1 or 2, wherein
the objective optical system is configured to optically relay a left eye measurement position where a pupil of a left eye of the examinee can be disposed, and to optically relay a right eye measurement position where a pupil of a right eye of the examinee can be disposed, and
the photographing unit includes:
a photographing unit for left eye including two or more cameras disposed so as to include a position, that is substantially conjugate optically to the left eye measurement position relayed by the objective optical system, within a field of vision; and
a photographing unit for right eye including two or more cameras disposed so as to include a position, that is substantially conjugate optically to the right eye measurement position relayed by the objective optical system, within a field of vision.

10. The ophthalmic apparatus of claim 9, wherein
the illumination optical system is configured to irradiate illumination light onto the left eye through the objective optical system, and to irradiate illumination light onto the right eye through the objective optical system, and
the photographic optical system includes:
a photographic optical system for left eye configured to receive returning light of the illumination light from the left eye through the objective optical system; and
a photographic optical system for right eye configured to receive returning light of the illumination light from the right eye through the objective optical system.

11. The ophthalmic apparatus of claim 10, wherein
the illumination optical system is configured to sequentially irradiate illumination light onto the left eye and the right eye through the objective optical system.

12. The ophthalmic apparatus of claim 9, wherein
the objective optical system includes two concave mirrors configured to be capable of rotating around a predetermined rotation axis, and
the two concave mirrors are configured to optically relay the left eye measurement position in a first rotational state around the predetermined rotation axis, and to optically relay the right eye measurement position in a second rotational state around the predetermined rotation axis.

13. The ophthalmic apparatus of claim 12, wherein
the two concave mirrors include:
a first ellipsoidal concave mirror; and
a second ellipsoidal concave mirror, wherein
a second focal point of the first ellipsoidal concave mirror is disposed at a first focal point of the second ellipsoidal concave mirror, and the left eye measurement position or the right eye measurement position can be disposed at a second focal point of the second ellipsoidal concave mirror, and
the ophthalmic apparatus further includes:
an optical path separating member for left eye disposed at a first focal point of the first ellipsoidal concave mirror in the first rotational state, and configured to separate an optical path of illumination light irradiated onto the left eye and an optical path of returning light of the illumination light from the left eye; and
an optical path separating member for right eye disposed at the first focal point of the first ellipsoidal concave mirror in the second rotational state, and configured to separate an optical path of illumination light irradiated onto the right eye and an optical path of returning light of the illumination light from the right eye.

14. The ophthalmic apparatus of claim 13, wherein
the illumination optical system includes an optical path switching member configured to guide illumination light to the optical path separating member for left eye or the optical path separating member for right eye.

15. The ophthalmic apparatus of claim 12, further comprising
a slide mechanism configured to move the objective optical system, the illumination optical system, the photographic optical system, and the photographing unit in a direction intersecting the predetermined rotation axis, in conjunction with a rotation of the two concave mirrors.

16. The ophthalmic apparatus of claim 1 or 2, wherein
the illumination optical system is configured to generate slit-shaped illumination light, and to deflect the slit-shaped illumination light, and
the photographic optical system is configured to receive the returning light in a light receiving range corresponding to an irradiated range of the slit-shaped illumination light on the eye.

17. The ophthalmic apparatus of claim 16, wherein
the photographic optical system is configured to capture light receiving result of the returning light using a rolling shutter system.
